# FASCICULE DE BREVET EUROPEEN

(11) **EP 2 084 125 B1**
(45) Date de publication et mention de la délivrance du brevet: **11.01.2012**
(21) Numéro de dépôt: 07866480.2
(22) Date de dépôt: 31.10.2007
(51) Int. Cl.: C07C 225/22, C07C 323/30, A61K 31/135, A61P 35/00

(54) **DERIVES D'AMINOBENZOCYCLOHEPTENE, LEURS PROCEDES DE PREPARATION ET LEUR UTILISATION EN THERAPEUTIQUE**
AMINOBENZOCYCLOHEPTENDERIVATE, VERFAHREN ZU DEREN HERSTELLUNG UND DEREN VERWENDUNG IN DER THERAPIE
AMINOBENZOCYCLOHEPTENE DERIVATIVES, METHODS FOR PREPARING THE SAME AND USES THEREOF IN THERAPY

(30) Priorité: 03.11.2006 FR 0609615
(43) Date de publication de la demande: 05.08.2009
(73) Titulaire: Universite De Haute Alsace, 68093 Mulhouse Cedex (FR); Université Louis Pasteur, 67070 Strasbourg Cedex (FR); Centre National de la Recherche Scientifique (CNRS), 75794 Paris Cedex 16 (FR)
(72) Inventeur: TARNUS-RONDEAU, Céline, 68170 Rixheim (FR); DEFOIN, Albert, 68440 Habsheim (FR); ALBRECHT, Sébastien, 68100 Mulhouse (FR); MAIEREANU, Anamaria, 67000 Strasbourg (FR); FAUX, Nadège, 67300 Schiltigheim (FR); PALE, Patrick, 67000 Strasbourg (FR)
(74) Mandataire: Oudin, Stéphane
(86) Numéro de dépôt international: PCT/FR2007/001812
(87) Numéro de publication internationale: WO 2008/059141

(56) Documents cités:
- WO-A-99/66934
- CELINE TARNUS ET AL: "3-Amino-2-tetralone Derivatives : Novel Potent and Selective Inhibitors of Aminopeptidase-M (EC 3.4.11.2)" ARCH. BIOCHEM. BIOPHYS., vol. 311, no. 1, 1994, pages 42-46, XP002444119 cité dans la demande
- DATABASE BEILSTEIN BEILSTEIN INSTITUTE FOR ORGANIC CHEMISTRY, FRANKFURT-MAIN, DE; XP002444121 Database accession no. 3751986 & CHEMICAL ABSTRACTS, vol. 54, no. 2, 1960, Columbus, Ohio, US; abstract no.: 3353,

## Description

La présente invention concerne des dérivés d'aminobenzocycloheptène, leurs procédés de préparation, et leur utilisation en thérapeutique.

Plus particulièrement, la présente invention concerne de nouveaux composés dérivés d'aminobenzocycloheptène à usage thérapeutique, dans les phénomènes relatifs à l'oncologie et plus particulièrement dans le processus d'angiogénèse.

L'angiogénèse est aujourd'hui un processus très étudié dans le domaine de la recherche contre le cancer. Des études (R.T. Poon et al, J. Cli. Oncol., 2001, 19, 1207-1225) montrent plus particulièrement que le processus de néoangiogénèse est indispensable au développement tumoral. Neutraliser ce processus dans le cas de cancers constitue donc une approche prometteuse pour la mise au point de nouveaux traitements anti-cancéreux permettant de lutter contre la progression tumorale, et qui soient moins toxiques que ceux utilisés jusqu'à présent.

Il a été montré récemment que l'aminopeptidase N, appelée par la suite AP-N, ou CD13, intervient dans les phénomènes de mobilité cellulaire. Elle est donc considérée comme un régulateur important de la morphogénèse endothéliale au cours du processus d'angiogénèse (H. Hashida et al, Gastroenterology, 2002, 122, 376-386; S.W. Bhagwat et al, Blood, 2001, 97, 652-659). Des études (R. Pasqualini et al, Cancer Res., 2000, 60, 722-727) montrent que les anticorps dirigés contre l'AP-N et son activité catalytique ou des inhibiteurs de faible poids moléculaire comme la bestatine ou l'actinonine présentent un effet négatif sur la croissance tumorale dans des modèles de souris. Cependant, la bestatine, l'actinonine ou l'amastatine sont des molécules inhibitrices peu sélectives. En effet, étant donné qu'il existe un grand nombre d'aminopeptidases qui sont structurellement très proches et qui agissent selon des mécanismes catalytiques très similaires, il est très difficile de concevoir des inhibiteurs sélectifs.

Une nouvelle série de molécules inhibitrices d'AP-N plus puissante et plus sélective a été découverte, la 3-amino-2-tétralone et ses dérivés (C. Schalk et al, Arch. Biochem. Biophys., 1994, 311(1), 42-46). Cette molécule, bien que présentant une grande sélectivité, présente l'inconvénient d'être peu stable en solution aqueuse.

Il est donc nécessaire de proposer de nouvelles molécules inhibitrices d'AP-N plus puissantes, plus sélectives et stables chimiquement.

A cet effet, et conformément à la présente invention, il est proposé de nouveaux composés dérivés d'aminobenzocycloheptène répondant à la formule générale (I): 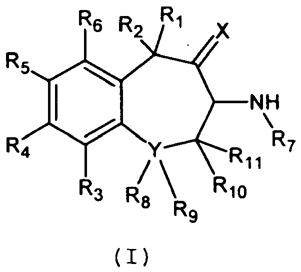 dans laquelle,
R₁ représente un atome d'hydrogène, de fluor, de chlore, de brome, un radical (C₁-C₆)alkyle, un radical (C₁-C₆) (cycloalkyl)alkyle, un radical (C₁-C₆) (hétérocycloalkyl) alkyle, un radical (C₁-C₆)aralkyle, un radical (C₁-C₆) hétéroaralkyle, un radical (C₁-C₆) alcoxy, un radical (C₁-C₆)aralkyloxy, un radical (C₁-C₆)alkylthio, un radical (C₁-C₆)aralkylthio;
R₂ représente un atome d'hydrogène, de fluor, de chlore, de brome, un radical (C₁-C₆)alkyle, un radical (C₁-C₆) (cycloalkyl)alkyle, un radical (C₁-C₆) (hétérocycloalkyl)alkyle, un radical (C₁-C₆)aralkyle, un radical (C₁-C₆)hétéroaralkyle; R₁ et R₂ pouvant former ensemble un cycle carboné non substitué ou substitué ou un hétérocycle non substitué ou substitué; ou R₁ pouvant être lié au cycle heptène par une double liaison, R₂ étant alors absent;
R₃, R₄, R₅ et R₆, identiques ou différents, représentent indépendamment l'un de l'autre un atome d'hydrogène, de fluor, de chlore, de brome, un radical (C₁-C₆)alkyle, un radical (C₁-C₆) (cycloalkyl)alkyle, un radical (C₁-C₆) (hétérocycloalkyl)alkyle, un radical polyfluoro (C₁-C₆)alkyle, un radical (C₁-C₆)aralkyle, un radical (C₁-C₆)hétéroaralkyle, un radical (C₁-C₆)alcoxy, un groupe aryle ou hétéroaryle; R₃ et R₄, R₄ et R₅, R₅ et R₆ indépendamment les uns des autres pouvant former ensemble un radical méthylènedioxy joignant les atomes de carbone adjacents ou un cycle carboné aromatique non substitué ou substitué ou un hétérocycle aromatique non substitué ou substitué;
R₇ représente un atome d'hydrogène, un radical (C₁-C₆)alkyle;
X est un atome d'oxygène, un atome de soufre, un radical imine N-R₁₂, un radical oxime N-O-R₁₃, dans lesquels R₁₂ et R₁₃ représentent un atome d'hydrogène, un radical (C₁-C₆)alkyle, un radical (C₁-C₆) (cycloalkyl) alkyle, un radical (C₁-C₆)(hétérocycloalkyl)alkyle, un radical (C₁-C₆)aralkyle, un radical (C₁-C₆)hétéroaralkyle;
Y est un atome de carbone; un atome d'azote, R₈ ou R₉ étant alors absent; un atome d'oxygène, un atome de soufre R₈ et R₉ étant alors absents;
R₈ et R₁₀, identiques ou différents, représentent indépendamment l'un de l'autre un atome d'hydrogène, de fluor, de chlore, de brome, un radical (C₁-C₆)alkyle, un radical (C₁-C₆) (cycloalkyl) alkyle, un radical (C₁-C₆)(hétérocycloalkyl) alkyle, un radical (C₁-C₆)aralkyle, un radical (C₁-C₆)hétéroaralkyle, un radical (C₁-C₆)alcoxy, un radical (C₁-C₆)aralkyloxy, un radical (C₁-C₆)alkylthio, un radical (C₁-C₆)aralkylthio;
R₉ et R₁₁, identiques ou différents, représentent indépendamment l'un de l'autre un atome d'hydrogène, de fluor, de chlore, de brome, un radical (C₁-C₆)alkyle, un radical (C₁-C₆) (cycloalkyl)alkyle, un radical (C₁-C₆)(hétérocycloalkyl)alkyle, un radical (C₁-C₆)aralkyle, un radical (C₁-C₆)hétéroaralkyle, un radical (C₁-C₆)alcoxy, un radical (C₁-C₆)aralkyloxy, un radical (C₁-C₆)alkylthio, un radical (C₁-C₆) aralkylthio, R₉ et R₁₁ pouvant former ensemble un cycle carboné non substitué ou substitué ou un hétérocycle non substitué ou substitué ou former une double liaison avec les deux atomes de carbone adjacents du cycle heptène;
ses isomères optiques et géométriques, notamment les formes énantiomères, ou diastéréoisomères et leurs mélanges, notamment les mélanges racémiques, ainsi que ses sels d'addition avec des acides minéraux et organiques, à l'exception du composé pour lequel R₄, R₅ et R₆ représentent un radical méthoxy, R₁, R₂, R₃, R₇, R₈, R₉, R₁₀, R₁₁ représentent un atome d'hydrogène, X représente un atome d'oxygène et Y représente un atome de carbone.

Ces acides sont avantageusement des acides pharmaceutiquement acceptables, mais d'autres acides peuvent être utilisés. Les acides sont par exemple les acides chlorhydrique, bromhydrique, iodhydrique, nitrique, sulfurique, phosphorique, acétique, mono- ou bi- ou trihalogénoacétique, formique, benzoïque, maléique, fumarique, succinique, tartrique, citrique, oxalique, glyoxylique, aspartique, alcanesulfonique, benzènesulfonique ou toluènesulfonique.

Dans la présente description, les radicaux alkyle, (cycloalkyl)alkyle, (hétérocycloalkyl)alkyle, aralkyle, hétéroaralkyle, alcoxy, aralkyloxy, alkylthio, aralkylthio sont linéaires ou ramifiés.

Pour les radicaux du type (C₁-C₆) (cycloalkyl) alkyle ou (C₁-C₆)aralkyle, (C₁-C₆) indique le nombre de carbone dans la partie alkyle.

Un groupe de composés préférés selon l'invention correspond aux composés pour lesquels R₁ représente un atome d'hydrogène, un atome de fluor, un radical (CH₂)ₙPh, un radical S(CH₂)ₙPh, n variant de 1 à 6, et de préférence de 1 à 5.

Un autre groupe de composés préférés selon l'invention correspond aux composés pour lesquels R₂ est un atome d'hydrogène.

Un autre groupe de composés préférés selon l'invention correspond aux composés pour lesquels X est un atome d'oxygène.

Un autre groupe de composés préférés selon l'invention correspond aux composés pour lesquels Y est un atome de carbone.

Un autre groupe de composés préférés selon l'invention correspond aux composés pour lesquels R₃, R₄, R₅ et R₆ identiques ou différents, représentent indépendamment l'un de l'autre un atome d'hydrogène, un atome de brome, un radical phényle, ou R₃ et R₄, R₄ et R₅, R₅ et R₆ indépendamment les uns des autres forment ensemble un cycle carboné aromatique non substitué ou substitué.

Un autre groupe de composés préférés selon l'invention correspond aux composés pour lesquels R₇ est un atome d'hydrogène.

Un autre groupe de composés préférés selon l'invention correspond aux composés pour lesquels simultanément Y est un atome de carbone et R₈, R₉, R₁₀, et R₁₁ sont des atomes d'hydrogène.

Les composés particulièrement préférés sont ceux pour lesquels R₂ et R₇ sont simultanément un atome d'hydrogène, X est un atome d'oxygène, et Y est un atome de carbone.

Parmi ces composés particulièrement préférés, un groupe de composés tout particulièrement préférés correspond aux composés pour lesquels R₁ est un atome d'hydrogène, un atome de fluor, un radical benzylthio, un radical (CH₂)ₙPh, où n=1-5.

Parmi ces composés particulièrement préférés, un autre groupe de composés tout particulièrement préférés correspond aux composés pour lesquels R₃, R₄, R₅, R₆ sont simultanément un atome d'hydrogène.

Parmi ces composés particulièrement préférés, un autre groupe de composés tout particulièrement préférés correspond aux composés pour lesquels R₄ et R₅ sont un atome d'hydrogène, et R₃ et R₆ représentent indépendamment l'un de l'autre un atome d'hydrogène, un atome de brome, un radical phényle, à la condition que R₃ et R₆ ne soient pas simultanément un atome d'hydrogène.

Parmi ces composés particulièrement préférés, un autre groupe de composés tout particulièrement préférés correspond aux composés pour lesquels R₃ et R₆ sont un atome d'hydrogène, et R₄ et R₅ représentent indépendamment l'un de l'autre un atome d'hydrogène, un atome de brome, un radical phényle, à la condition que R₄ et R₅ ne soient pas simultanément un atome d'hydrogène.

Parmi ces composés particulièrement préférés, un autre groupe de composés tout particulièrement préférés correspond aux composés pour lesquels R₃ et R₄, R₅ et R₆ indépendamment les uns des autres forment ensemble un cycle carboné aromatique, non substitué ou substitué, joignant les atomes de carbone adjacents.

Les composés selon l'invention particulièrement préférés répondent aux formules (Ia) à (Ie) ci-dessous: 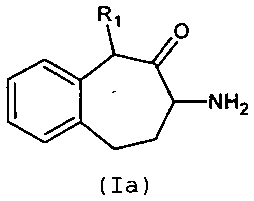 dans laquelle R₁ représente un atome d'hydrogène, un atome de fluor, le radical CH₂Ph, le radical (CH₂)₂Ph, le radical (CH₂)₃Ph, le radical (CH₂)₄Ph, le radical (CH₂)₅Ph, le radical S-CH₂Ph, le radical =CH-Ph; 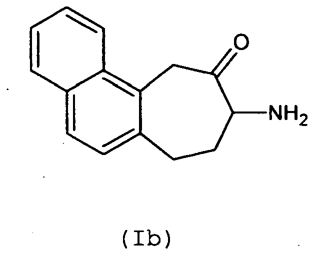 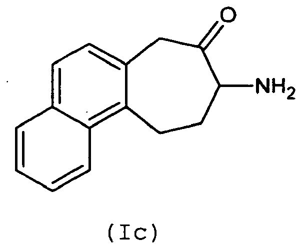 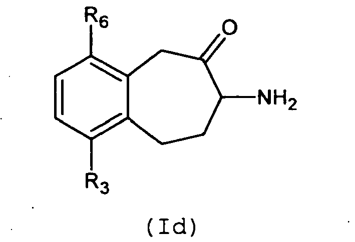 dans laquelle les substituants R₃ et R₆ sont tels que définis dans les tableaux I et II suivants:

**Tableau I**

| R₃ | R₆ |
|---|---|
| H | phényle |
| H | Br |
| Phényle | H |
| Br | H |
| Phényle | Br |
| Br | phényle |
| phényle | phényle |
| Br | Br |

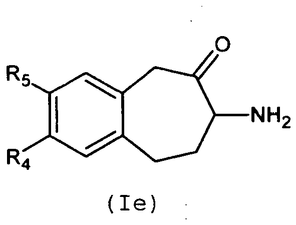

**Tableau II**

| R₄ | R₅ |
|---|---|
| H | phényle |
| H | Br |
| Phényle | H |
| Br | H |
| phényle | phényle |
| Br | Br |

Il est bien entendu que tous les isomères optiques et géométriques, notamment les formes énantiomères, ou diastéréoisomères et leurs mélanges, notamment les mélanges racémiques, ainsi que les sels d'addition avec des acides minéraux et organiques des composés décrits ci-dessus appartiennent à la présente invention.

La présente invention concerne également un procédé de préparation des composés de formule (I) pour lesquels R₁ représente un atome d'hydrogène, un atome de fluor, un radical (CH₂)ₙPh, le radical =CH-Ph, R₂ est un atome d'hydrogène ou absent, R₇, R₈, R₉, R₁₀, et R₁₁ sont des atomes d'hydrogène; X est un atome d'oxygène, un radical NOH, Y est un atome de carbone, R₃, R₄, R₅, R₆ ont la signification déjà indiquée; et ses sels, dans lequel
1) on introduit sur le composé de formule générale (II) 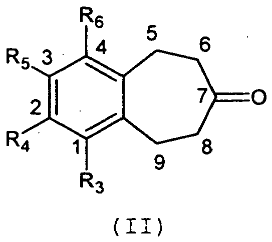 une fonction amine protégée -NHPG en position 7 par réaction de la fonction cétone, où PG est un groupe protecteur, et en position 6 une fonction cétone lorsque X est un atome d'oxygène ou une fonction cétone-oxime lorsque X est le radical NOH pour former un dérivé de formule générale (III) 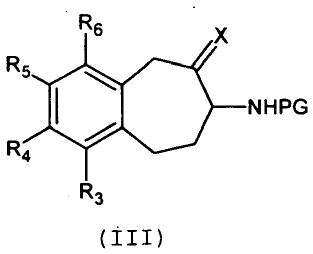
2) lorsque R₁ n'est pas un atome d'hydrogène, on introduit la fonction correspondant à R₁ en position 5 pour former un dérivé de formule générale (IV) 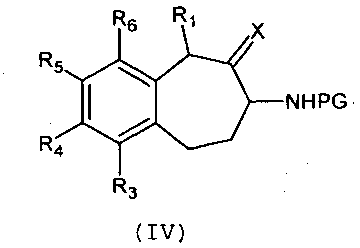
3) on déprotège la fonction amine NH-PG par clivage du groupe PG.

La fonction amine en position 7 à partir de la fonction cétone peut être obtenue par réaction de condensation de la fonction cétone avec une amine primaire suivie d'une réduction par NaBH₄ par exemple.

Dans le cadre de la présente invention, on appelle groupe protecteur PG un groupe qui permet d'une part de protéger la fonction réactive amine pendant la synthèse des composés et d'autre part de régénérer cette fonction réactive intacte en fin de synthèse. Un tel groupe protecteur est par exemple un *N-tert*-butoxycarbonyl(Boc), la déprotection se faisant par hydrolyse acide en présence par exemple d'acide chlorhydrique.

La fonction cétone en position 6 lorsque X est un atome d'oxygène peut être obtenue par introduction d'un groupe hydroxyle en position 6 suivie d'une oxydation par exemple avec le periodinane de Dess-Martin.

La présente invention concerne également un procédé de préparation des composés de formule (I) pour lesquels R₁ représente un radical S(CH₂)ₙPh, R₂ est un atome d'hydrogène, R₇, R₈, R₉, R₁₀, et R₁₁ sont des atomes d'hydrogène; X est un atome d'oxygène, Y est un atome de carbone, R₃, R₄, R₅, R₆ ont la signification déjà indiquée; et ses sels, dans lequel
1) on forme sur le composé de formule générale (II) 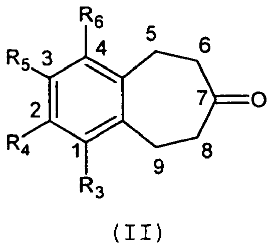 une double liaison entre les positions 5 et 6
2) on introduit une fonction amine protégée -NHPG en position 7 par réaction de la fonction cétone, où PG est un groupe protecteur pour former un dérivé de formule générale (V) 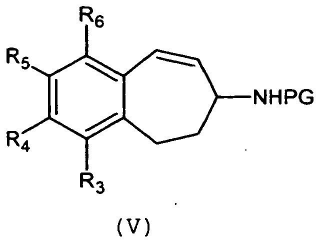
3) on oxyde la double liaison pour former une fonction époxyde liant les atomes de carbone en positions 5 et 6
4) on introduit le radical S(CH₂)ₙPh en position 5 pour former un dérivé de formule générale (VI) 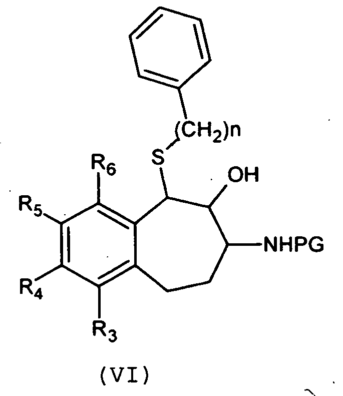
5) on oxyde la fonction alcool du dérivé obtenu
6) on déprotège la fonction amine NH-PG par clivage du groupe PG.

Les conditions opératoires utilisées dans les différentes étapes décrites ci-dessus sont classiques pour l'Homme du métier.

Le composé de formule générale (II) est décrit dans la littérature où:
- R₆, R₄, R₃ représentent un atome d'hydrogène et R₅ représente un radical CF₃, CH₃, C(CH₃)₃, ou OCH₃
- R₅ et R₄ représentent un atome d'hydrogène et R₆ et R₃ représentent un radical OCH₃
- R₅ et R₄ représentent un atome d'hydrogène et R₆ et R₃ représentent un radical CH₃
- R₅ et R₄ représentent un atome de brome et R₆ et R₃ représentent un radical OCH₃
- R₆ et R₃ représentent un atome d'hydrogène et R₅ et R₄ représentent un radical OCH₃.

Comme on le verra dans les exemples ci-après, les composés selon la présente invention sont des inhibiteurs d'AP-N plus sélectifs que les inhibiteurs connus à ce jour, avec une constante d'inhibition Ki inférieure à 10⁻⁵M et supérieure à 10⁻³M avec d'autres aminopeptidases.

La présente invention concerne également une composition pharmaceutique, contenant à titre de principe actif un composé de formule (I) tel que décrit ci-dessus, ou un de ses sels d'addition avec des acides minéraux et organiques pharmaceutiquement acceptables, étant entendu que le composé pour lequel R₄, R₅ et R₆ représentent un radical méthoxy, R₁, R₂, R₃, R₇, R₈, R₉, R₁₀, R₁₁ représentent un atome d'hydrogène, X représente un atome d'oxygène et Y représente un atome de carbone, n'est pas exclu. Ces compositions comprennent une dose efficace d'un de ces composés, ou d'un sel pharmaceutiquement acceptable, et le principe actif peut être éventuellement mélangé à au moins un excipient pharmaceutiquement acceptable. Ces excipients sont connus de l'Homme du métier et seront adaptés à la forme pharmaceutique et au mode d'administration souhaité.

Les compositions pharmaceutiques selon l'invention se présentent sous toutes les formes connues de l'Homme du métier, adaptées notamment pour une administration par voie orale, sublinguale, intramusculaire, intraveineuse, topique, locale, intranasale, transdermique ou rectale. Les compositions pharmaceutiques peuvent ainsi se présenter sous la forme de gélules, de comprimés, de granulés, de suppositoires, de préparations injectables, de crèmes, préparés selon des méthodes usuelles.

L'invention concerne également l'utilisation d'un composé de formule (I) selon l'invention, étant entendu que le composé pour lequel R₄, R₅ et R₆ représentent un radical méthoxy, R₁, R₂, R₃, R₇, R₈, R₉, R₁₀, R₁₁ représentent un atome d'hydrogène, X représente un atome d'oxygène et Y représente un atome de carbone, n'est pas exclu, pour la préparation d'un médicament destiné au traitement des cancers, des tumeurs, et notamment pour traiter et prévenir les maladies impliquant l'inhibition des métalloprotéases, plus particulièrement l'Aminopeptidase-N.

Les exemples suivants illustrent la présente invention sans toutefois en limiter la portée.

### PREPARATIONS

### Préparation 1: 1-bromo-2,3-bis-bromométhyl-benzène

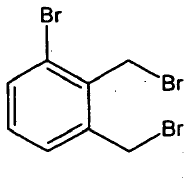

On irradie une solution de 3-bromo-*o*-xylène (2 g, 10.8 mmoles) et de *N*-bromosuccinimide (4,04 g, 22.7 mmoles) dans du tétrachlorure de carbone (70 mL) par une lampe au mercure HPK125 pendant 2 heures. On dilue le mélange réactionnel à l'acétate d'éthyle, puis on lave au chlorure d'ammonium aqueux (2 M) et on sèche sur sulfate de magnésium. On évapore le solvant pour obtenir 3,7 g d'une huile incolore.

RMN ¹H (CDCl₃):7.56 (d, J=8.1 Hz, 1 Har); 7.31 (d, J=7.5 Hz, 1 Har); 7.15 (t, J=7.8 Hz, 1 Har); 4.84 (s, *CH₂*Br) ; 4.64 (s, *CH₂*Br)

Ce composé est utilisé à la place de 1'α,α'-dibromo-*o*-xylène utilisé dans la préparation 2 lorsque l'on veut synthétiser un composé ayant comme substituant R₃ ou R₆ un atome de brome.

### Préparation 2: 7-oxo-5,6,8,9-tétrahydrobenzocycloheptène-6,8-dicarboxylate de diméthyle

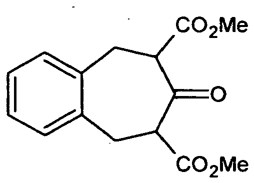

On chauffe un mélange de α,α'-dibromo-o-xylène (51 g, 189 mmoles), de 1,3-acétonedicarboxylate de diméthyle (49,3 g, 283 mmoles), de bromure de tétrabutylammonium (38,3 g, 118,8 mL), dans une solution aqueuse d'hydrogéhocarbonate de sodium 1 N (1 L) et de dichlorométhane (400 mL) à 40°C sous argon pendant une nuit sous agitation vigoureuse. La phase organique est séparée et évaporée à sec. Le résidu est dilué à l'acétate d'éthyle, lavé à la saumure (4 x 100 mL), et séché sur sulfate de magnésium. On obtient une résine jaune (80 g) utilisée sans aucune autre purification.

Point de Fusion=100-110°C

Isomère trans majoritaire, RMN ¹H (CDCl₃): 7.23 (m, 4 Har); 3.93 (dd, H-C(6), H-C(8)); 3.70 (s,COOMe); 3.23 (dd, Ha-C(5),Ha-C(9)); 3.14 (m, Hb-C(5), Hb-C(9)); J(5a,5b)=15.0 Hz, J(5a,6)=9.0 Hz, J(5b,6)=3.5 Hz
Isomère cis minoritaire, RMN ¹H (CDCl₃): 7.26 (m, 4 Har); 3.79 (s,COOMe); 3.56 (dd, H-C(6),H-C(8)); 3.22 (m, Ha-C(5),Ha-C(9)); 3.15 (m, Hb-C(5),Hb-C(9)); J(5a,5b)=14.8 Hz, J(5a,6)=11.3 Hz, J(5b,6)=3.3 Hz

### Préparation 3: 5,6,8,9-tétrahydro-benzocycloheptèn-7-one (composé répondant à la formule II)

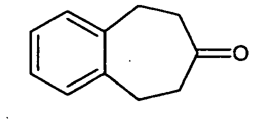

Le mélange isomérique de 7-oxo-5,6,8,9-tétrahydrobenzocycloheptène-6,8-dicarboxylate de diméthyle (80 g) obtenu selon la préparation 2 est laissé à reflux dans une solution aqueuse d'acide sulfurique 3 M (300 mL) et d'acétonitrile (50 mL) pendant une nuit sous argon. On dilue le mélange à l'éther diéthylique, neutralise avec une solution aqueuse d'hydroxyde de sodium 2 M (3 x 300 mL), sèche sur sulfate de magnésium et évapore à sec. On distille le résidu à 97-98°C sous 0.4-0.5 Torr pour obtenir des cristaux incolores (26,1 g, 84% à partir de l'α,α'-dibromo-*o*-xylène).

Point de fusion: 43-44°C

RMN ¹H (CDCl₃) : 7.23 (m, 4 Har); 2.91 (m, 2 H-C(6), 2 H-C(8)); 2.62 (m, 2 H-C(5),2 H-C(9)).

### Préparation 4: 7-(tert-butoxycarbonyl-amino)-6,7,8,9-tétrahydro-5H-benzocycloheptèn-6-ol

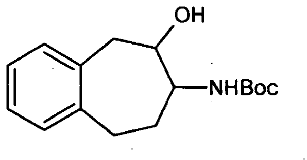

Boc = *tert*-butoxycarbonyl

A une solution de 5,6,8,9-tétrahydro-benzocycloheptèn-7-one (1,07 g, 6,68 mmoles) (préparation 3), de triéthylamine (1,3 mL, 9,35 mmoles), dans du toluène anhydre (15 mL) on ajoute goutte à goutte du trifluorométhanesulfonate de triméthylsilyle (1,45 mL, 8,01 mmoles) à température ambiante sous argon. On chauffe le milieu réactionnel à 90°C pendant 2 heures, on dilue au cyclohexane, lave à une solution aqueuse de chlorure d'ammonium 2 M et à la saumure. On sèche la phase organique sur sulfate de magnésium et on évapore pour obtenir l'éther de silylénol qui est utilisé sans autre purification.

A un mélange d'éther de silylénol (6.68 mmoles) dans du dichlorométhane anhydre (20 mL) on ajoute par portions de l'acide 3-chloroperoxybenzoïque (1.4 g, 8.01 mmoles) à 0°C sous argon. On agite le milieu réactionnel à 0°C pendant 2 heures, on filtre le précipité d'acide 3-chlorobenzoïque et on évapore le filtrat pour obtenir l'hydroxy-cétone qui est utilisée sans autre purification.

On agite un mélange d'hydroxy-cétone (6.68 mmoles), d'isopropoxyde de titane (IV) (4 mL, 13.3 mmoles) et d'ammoniac saturé dans l'éthanol (20 mL) sous argon à température ambiante pendant une nuit. On ajoute ensuite du borohydrure de sodium (380 mg, 10 mmoles) et on agite le mélange obtenu à température ambiante pendant 2 heures. On évapore les solvants, on dilue le résidu à l'acétate d'éthyle et on ajoute de l'hydroxyde d'ammonium aqueux 1 N (20 mL). On filtre le précipité inorganique obtenu, on le lave avec un mélange 1/1 d'acétate d'éthyle et d'hydroxyde d'ammonium aqueux 1 N (3 x 20 mL). On sépare la phase organique et on extrait la phase aqueuse restante à l'acétate d'éthyle (3 x 20 mL). On sèche les extraits organiques combinés sur sulfate de magnésium, on concentre à sec pour obtenir l'amino-alcool brut.

On agite un mélange de cet amino-alcool (6.68 mmoles), de dicarbonate de di-*tert*-butyle (3.2 g, 14.6 mmoles) et de carbonate de sodium (780 mg, 7.34 mmoles) dans du méthanol (10 mL) sous argon pendant une nuit. On évapore le solvant, on lave le solide obtenu à l'eau (3 x 20 mL) et à l'éther d'isopropyle froid (3 fois) pour obtenir le 7-(*tert*-butoxycarbonyl-amino)-6,7,8,9-tétrahydro-5*H*-benzocycloheptèn-6-ol sous forme de cristaux incolores (985 mg, 53% à partir du 5,6,8,9-tétrahydro-benzocycloheptèn-7-one).

Point de fusion: 178-180°C

### Préparation 5: 7-(benzyloxycarbonyl-amino)-6,7,8,9-tétrahydro-5H-benzocycloheptèn-6-ol

On reproduit la synthèse décrite selon la préparation 4 en utilisant 3 g (18.7 mmoles) de 5,6,8,9-tétrahydro-benzocycloheptèn-7-one (préparation 3).

Une fois l'amino-alcool obtenu, on agite un mélange de cet amino-alcool (18.7 mmoles), de chloroformiate de benzyle (3.8 mL, 26.2 mmoles) dans du THF (40 mL) et avec du carbonate de sodium (5.6 g, 52.4 mmoles) sous argon pendant une nuit à température ambiante. On dilue le milieu réactionnel à l'acétate d'éthyle et on lave au chlorure d'ammonium aqueux 2 M, à la saumure, on sèche sur sulfate de magnésium. On évapore le solvant, on lave le solide obtenu à l'éther d'isopropyle pour obtenir le 7-(benzyloxycarbonyl-amino)-6,7,8,9-tétrahydro-5*H*-benzocycloheptèn-6-ol sous forme de cristaux incolores (3 g, 52% à partir de 5,6,8,9-tétrahydro-benzocycloheptèn-7-one).

Point de fusion: 148-150°C

### Préparation 6: 7-(tert-butoxycarbonyl-amino)-5,7,8,9-tétrahydro-benzocycloheptèn-6-one

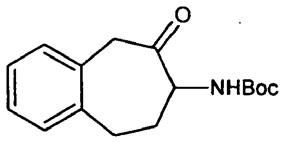

Ce composé correspond à un dérivé de formule (III).

A une solution de 7-(*tert*-butoxycarbonyl-amino)-6,7,8,9-tétrahydro-5*H*-benzocycloheptèn-6-ol (préparation 4) (1 g, 3.6 mmoles) dans du dichlorométhane (20 mL) on ajoute du DMP (periodinane de Dess-Martin) (2.3 g, 5.4 mmoles) et on agite le mélange à température ambiante sous argon pendant 3 heures. On dilue le milieu réactionnel à l'acétate d'éthyle (50 mL), on ajoute du thiosulfate de sodium pentahydraté (6.7 g, 27 mmoles, 5 eq.) et une solution aqueuse d'hydrogénocarbonate de sodium 1 N, et on agite à température ambiante pendant 1 heure. On lave la phase organique plusieurs fois à une solution aqueuse d'hydrogénocarbonate de sodium 1 N et à la saumure, et on sèche sur sulfate de magnésium. On évapore le solvant, et le solide obtenu est lavé à l'éther d'isopropyle (3 fois) pour obtenir des cristaux incolores de 7-(*tert-*butoxycarbonyl-amino)-5,7,8,9-tétrahydro-benzocycloheptèn-6-one (765 mg, 77%).

Point de fusion: 150-152°C

RMN ¹H (CDCl₃): 7.18 (m, 4 Har); 5.43 (d, NH); 4.55 (dt, H-C(7)); 3.95 (d, Ha-C(5)); 3.60 (d, Hb-C(5)); 3.03 (ddd, Ha-C(9)); 2:89 (ddd, Hb-C(9)); 2.63 (dddd, Ha-C(8)); 1.46 (m, Hb-C(8)); 1.43 (s, tBu); J(5a,5b)=14.6 Hz, J(7,NH)=ca 7.0 Hz, J(7,8a)=7.0 Hz, J(7,8b)=11.3 Hz, J(8a,8b)=12.8 Hz, J(8a,9a)=9.0 Hz, J(8a,9b)=3.4 Hz, J(8b,9a)=3.4 Hz, J(8b,9b)=9.0 Hz, J(9a,9b)=14.6 Hz.

### Préparation 7: 7-(benzyloxycarbonyl-amino)-5,7,8,9-tétrahydro-benzocycloheptèn-6-one

On reproduit la synthèse décrite selon la préparation 6 en utilisant le 7-(benzyloxycarbonyl-amino)-6,7,8,9-tëtrahydro-5*H*-benzocycloheptèn-6-ol synthétisé selon la préparation 5 (2.71 g, 8.7 mmoles) et le DMP (5.2 g, 12.2 mmoles) pour obtenir des cristaux incolores de 7-(benzyloxycarbonyl-amino)-5,7,8,9-tétrahydro-benzocycloheptèn-6-one (2.48 g, 92%).

Point de fusion: 119-121°C

RMN ¹H (CDCl₃) : 7.35-7.30 (m, 5 Har); 7.22-7.15 (m, 4 Har); 5.71 (d, NH); 5.08 (s1, OBn); 4.61 (m, H-C(7)); 3.97 (d, Ha-C(5)); 3.61 (d, Hb-C(5)); 3.06 (ddd, Ha-C(9)); 2.90 (ddd, Hb-C(9)); 2.68 (m, Ha-(C8)); 1.50 (m, Hb-C(8)). J(5a,5b)=14.2 Hz, J(7,NH)=ca. 6.0 Hz, J(7,8a)=7.6 Hz, J(7,8b)=11.6 Hz, J(8a,9a)=9.2 Hz, J(8a,9b)=3.2 Hz, J(8a,8b)=11.4 Hz, J(8b,9a)=3.0 Hz, J(8b,9b)=8.8 Hz, J(9a,9b)=14.6 Hz.

### EXEMPLES

### Exemple 1: chlorhydrate du 7-amino-5,7,8,9-tétrahydro-benzocycldheptèn-6-one oxime

A une solution de 5,6,8,9-tétrahydro-benzocycloheptèn-7-one (préparation 3, 1.0 g, 6.24 mmoles) dans de l'acide chlorhydrique sec 2 N dans du méthanol (13 mL), on ajoute du nitrite de *n*-butyle (1.1 mL, 9.3 mmmoles) sous argon à 0°C. On agite le mélange réactionnel à 0°C pendant 45 minutes, et on hydrolyse à l'hydrogénocarbonate de sodium aqueux 1 N. Après extraction à l'éther éthylique, la phase organique est lavée à l'hydrogénocarbonate de sodium aqueux 1 N, puis à l'eau et séchée sur sulfate de magnésium. Après évaporation des solvants, on lave le solide obtenu à l'isopropanol pour obtenir le 7,7-diméthoxy-5,7,8,9-tétrahydro-benzocycloheptèn-6-one oxime (903 mg, 62%).

On agite sous argon à 0°C pendant 15 minutes une solution de 7,7-diméthoxy-5,7,8,9-tétrahydro-benzocycloheptèn-6-one oxime (1.0 g, 4.26 mmoles), d'acide chlorhydrique aqueux 6 N (18 mL) et d'éther éthylique (18 mL). On extrait le mélange réactionnel à l'éther éthylique, on lave la solution organique à l'hydrogénocarbonate de sodium aqueux 1 N puis à l'eau et on sèche sur sulfate de magnésium. On évapore le solvant et on purifie le résidu par chromatographie (cyclohexane/acétate d'éthyle, 7/3 à 5/5) pour obtenir le 8,9-dihydro-5*H*-benzocycloheptène-6,7-dione 6-oxime (694 mg, 86%).

On agite sous argon à température ambiante pendant 6 heures une solution de 8,9-dihydro-5*H*-benzocycloheptène-6,7-dione 6-oxime (2.32 g, 12.3 mmoles) et de benzylamine (1.35 mL, 12.4 mmoles) dans de la pyridine (7 mL). On dilue la solution au méthanol (7 mL), on ajoute du borohydrure de sodium (0.55 g, 14.5 mmoles), on agite le mélange réactionnel à température ambiante pendant 1 heure. Après dilution à l'éther éthylique, la phase organique est lavée à l'hydrogénocarbonate de sodium aqueux 1 N puis à l'eau et séchée sur sulfate de magnésium. Après évaporation des solvants, on lave le solide obtenu à l'éther diisopropylique pour obtenir des cristaux blanc crème de 7-benzylamino-5,7,8,9-tétrahydro-benzocycloheptèn-6-one oxime (3.18 g, 93%), correspondant à un composé de formule III, avec X étant le radical NOH.

Point de fusion: 144-146°C (*i*Pr₂O)

RMN ¹H (CDCl₃) : 7.32 (m, 6 Har); 7.14 (m, 3 Har); 3.95 (d, Ha-C(5)); 3.81 (d, Ha-C(NBn)); 3.72 (d, Hb-C(5)); 3.67 (d, Hb-C(NBn)); 3.50 (dd, H-C(7)); 3.14 (m, Ha-C(9)); 2.65 (m, Hb-C(9)); 2.07 (m, Ha-C(8)); 1.85 (m, Hb-C(8)); J(NCH₂Ph)=12.9 Hz, J(5a,5b)=14.3 Hz, J(7,8a)=4.8 Hz, J(7,8b)=6.4 Hz, J(8a,8b)=13.6 Hz, J(8a,9a)=10.8 Hz, J(8a,9b)=2.9 Hz, J(8b,9a)=2.6 Hz, J(8b,9b)=7.2 Hz, J(9a,9b)=14.4 Hz.

On obtient le chlorhydrate correspondant en déprotégeant la fonction amine selon la méthode ci-dessous:
On hydrogénolyse le 7-benzylamino-5,7,8,9-tétrahydro-benzocycloheptèn-6-one oxime obtenu (100 mg, 0.356 mmole) dans l'éthanol (3 mL) et l'acide chlorhydrique aqueux 1 N (357 µL, 0.357 mmole) en présence de palladium à 5% sur charbon (7 mg) sous hydrogène (1 atm) à température ambiante pendant 13 heures. On élimine le catalyseur par centrifugation et on évapore le solvant. On recristallise le composé obtenu dans du 2-propanol/éther diéthylique pour obtenir des cristaux beiges de chlorhydrate de 7-amino-5,7,8,9-tétrahydro-benzocycloheptèn-6-one oxime (60 mg, 75%).

Point de fusion: 270-280°C

RMN ¹H (CD₃OD): 7.26-7.18 (m, 4 Har); 4.23 (d, Ha-C(5)); 4.01 (dd, H-C(7)); 3.42 (d, Hb-C(5)); 3.04 (ddd, Ha-C(9)); 2.93 (ddd, Hb-C(9)); 2.41 (m, Ha-C(8)); 1.66 (m, Hb-C(8)); J(5a,5b)=15.2 Hz, J(7,8a)=5.4 Hz, J(7,8b)=11.6 Hz, J(8a,8b)=12.6 Hz, J(8a,9a)=3.4 Hz, J(8a,9b)=8.6 Hz, J(8b,9a)=9.0 Hz, J(8b,9b)=3.4 Hz, J(9a,9b)=14.6 Hz.

### Exemple 2: chlorhydrate de 7-amino-5,7,8,9-tétrahydro-benzocycloheptèn-6-one

Cette étape correspond à la déprotection de la fonction amine des dérivés de formule (III) ou de formule (IV).

Un mélange de 7-(*tert*-butoxycarbonyl-amino)-5,7,8,9-tétrahydro-benzocycloheptèn-6-one (composé de formule (III), préparation 6) (765 mg, 2.78 mmoles) et de chlorure d'hydrogène 2.2 M dans l'éther diéthylique (5 mL) dans du dioxane (5 mL) est agité à température ambiante sous argon pendant 72 heures. Le solide obtenu est filtré et recristallisé dans un mélange 2-propanol/éther diéthylique pour obtenir des cristaux incolores de chlorhydrate de 7-amino-5,7,8,9-tétrahydro-benzocycloheptèn-6-one (500 mg, 855%).

Point de fusion: 230-240°C (déc.)

RMN ¹H (CDCl₃): 7.25 (m, 4 Har); 4.38 (dd, H-C(7)); 4.22 (d, Ha-C(5)); 3.63 (d, Hb-C(5)); 3.26 (ddd, Ha-C(9)); 3.04 (ddd, Hb-C(9)); 2.54 (m, Ha-C(8)); 1.69 (m, Hb-C(8)); J(5a,5b)=13.8 Hz, J(7,8a)=6.8 Hz, J(7,8b)=12.1 Hz, J(8a,8b)=12.8 Hz, J(8a,9a)=2.8 Hz, J(8a,9b)=8.2 Hz, J(8b,9a)=10.0 Hz, J(8b,9b)=3.0 Hz, J(9a,9b)=14.8 Hz.

### Exemple 3: chlorhydrate de 7-amino-5-fluoro-5,7,8,9-tétrahydro-benzocycloheptèn-6-one

On prépare une solution d'hexaméthyldisilazane de lithium (1.6 mmoles) à partir de *n*-butyl lithium 1.6 M (1 mL, 1.6 mmoles) dans l'hexane et d'hexaméthyldisilazane (340 µL, 1.6 mmoles) agités sous argon à -78°C pendant 15 minutes.

A cette solution, on ajoute goutte à goutte une solution de 7-(*tert*-butoxycarbonyl-amino)-5,7,8,9-tétrahydro-benzocycloheptèn-6-one (préparation 6) (200 mg, 0.73 mmole) et d'hexaméthylphosphoramide (380 µL, 2.18 mmoles) dans du tétrahydrofurane anhydre (4 mL) à -78°C. On agite le milieu réactionnel sous argon à -78°C pendant 0.5 heure, puis on ajoute du chlorure de *tert*-butyldiméthylsilyle (263 mg, 1.74 mmoles) et le milieu réactionnel obtenu est agité à -78°C pendant encore 15 minutes, dilué à l'éther diéthylique et hydrolysé par du chlorure d'ammonium aqueux 2 M, lavé à la saumure, et séché sur sulfate de magnésium. On évapore les solvants et l'éther d'énol silylé est utilisé brut sans autre purification.

A une solution d'éther d'énol silylé dans un mélange d'acétonitrile (8 mL) et d'hydrogénocarbonate de sodium aqueux 1 M (2 mL) on ajoute du Selectfluor (309 mg, 0.87 moles) sous argon à 0°C. On agite le milieu réactionnel à 0°C pendant 0.5 heure, on dilue à l'acétate d'éthyle et on hydrolyse au chlorure d'ammonium aqueux 2 M, on lave à la saumure et on sèche sur sulfate de magnésium. Après évaporation à sec, on dissout le résidu dans du tétrahydrofurane (10 mL) et on ajoute du fluorure de tétrabutylammonium (76 mg, 0.29 mmole). On agite le milieu réactionnel sous argon à 0°C pendant 10 minutes, on dilue à l'acétate d'éthyle, hydrolyse au chlorure d'ammonium aqueux 2 M, lave à la saumure et sèche sur sulfate de magnésium. On évapore le solvant et on purifie le résidu par chromatographie (cyclohexane/acétate d'éthyle, 8/2) pour obtenir des cristaux incolores de 7-(*tert*-butoxycarbonyl-amino)-5-fluoro-5,7,8,9-tétrahydro-benzocycloheptèn-6-one (110 mg, 52%).

Point de fusion: 127-129°C

RMN ¹H (CDCl₃) : 7.30 (m, 4 Har); 5.57 (d, H-C(5)); 5.50 (d, NH); 5.27 (m, H-C(7)); 3.50 (t, Ha-C(9)); 2.77 (m, Hb-C(9)); 2.67 (m, Ha-C(8)); 1.55 (m, Hb-C(8)); 1.46 (s, tBu); J(5,F)=50.0 Hz, J(7,NH)=6.0 Hz, J(7,8a)=12.0 Hz, J(7,8b)=6.0 Hz, J(8a,8b)=13.1 Hz, J(8a,9b)=J(8b,9a)=6.0 Hz, J(9a,9b)=14.0 Hz.

On obtient le chlorhydrate correspondant en déprotégeant la fonction amine selon l'exemple 2.

### Exemple 4: chlorhydrate de 7-amino-5-benzylidène-5,7,8,9-tétrahydro-benzocycloheptèn-6-one

A une solution d'hexaméthyldisilazane de lithium (0.8 mmoles) (préparé à partir de *n*-butyl lithium 1.6 M (0.5 mL, 0.8 mmoles, 2.2 eq.) dans l'hexane et d'hexaméthyldisilazane (170 µL, 0.8 mmoles, 2.2 eq.) agitée sous argon à -78°C pendant 15 minutes) on ajoute goutte à goutte une solution de 7-(*tert*-butoxycarbonyl-amino)-5,7,8,9-tétrahydro-benzocycloheptèn-6-one (préparation 6) (100 mg, 0.36 mmole) et d'hexaméthylphosphoramide (190 µL, 1.1 mmoles, 3 eq.) dans du tétrahydrofurane anhydre (3 mL) à -78°C. On agite le milieu réactionnel sous argon à -78°C pendant 20 minutes, puis on ajoute une solution de benzaldéhyde (74 µL, 0.73 mmole, 2 eq.) dans du tétrahydrofurane (2 mL) et le milieu réactionnel obtenu est agité à -78°C pendant encore 1 heure puis pendant 2.5 heures à température ambiante. On dilue le milieu réactionnel à l'acétate d'éthyle et on hydrolyse au chlorure d'ammonium aqueux 2 M, lave à la saumure, et sèche sur sulfate de magnésium. On évapore les solvants et on purifie le résidu par chromatographie (cyclohexane/acétate d'éthyle, 7/3) pour obtenir des cristaux incolores de 5-benzylidène-7-(*tert-*butoxycarbonyl-amino)-5,7,8,9-tétrahydro-benzocycloheptèn-6-one (70 mg, 53%).

Point de fusion: 156-158°C

RMN ¹H (CDCl₃): 7.95 (s, H-C(1')); 7.29-7.09 (m, 9 Har); 5.49 (d1, NH); 4.45 (ddd, H-C(7)); 2.99 (ddd, Ha-C(9)); 2.76 (ddd, Hb-C(9)); 2.56 (dddd, Ha-C(8)); 1.74 (m, Hb-C(8)); 1.41 (s, *t*Bu); J(7,NH)=ca 7.0 Hz, J(7,8a)=8.6 Hz, J(7,8b)=10.4 Hz, J(8a,8b)=12.6 Hz, J(8a,9a)=13.0 Hz, J(8a,9b)=7.4 Hz, J(8b,9a)=7.6 Hz, J(8b,9b)=1.2 Hz, J(9a,9b)=13.8 Hz.

On obtient le chlorhydrate correspondant en déprotégeant la fonction amine selon l'exemple 2 en utilisant 20 mg (55 µmoles) de 5-benzylidène-7-(*tert-*butoxycarbonyl-amino)-5,7,8,9-tétrahydro-benzocycloheptèn-6-one et du chlorure d'hydrogène 2.2 M dans de l'éther diéthylique (0.5 mL) dans du dioxane (0.5 mL). On obtient 13 mg (79%) de chlorhydrate de 7-amino-5-benzylidène-5,7,8,9-tétrahydr.o-benzocycloheptèn-6-one.

RMN ¹H (CD₃OD): 8.05 (s, H-C(1')); 7.46-7.39 (m, 2 Har); 7.32-7.13 (m, 7 Har); 4.00 (dd, H-C(7)); 3.09 (ddd, Ha-C(9)); 2.96 (ddd, Hb-C(9)); 2.45 (dddd, Ha-C(8)); 2.08 (dddd, Hb-C(8)); J(7,8a)=8.2 Hz, J(7,8b)=11.0 Hz, J(8a,8b)=13.0 Hz, J(8a,9a)=12.6 Hz, J(8a,9b)=7.4 Hz, J(8b,9a)=7.6 Hz, J(8b,9b)=1.2 Hz, J(9a,9b)=14.2 Hz.

### Exemple 5: chlorhydrate de 7-amino-5-benzyl-5,7,8,9-tétrahydro-benzocycloheptèn-6-one

On utilise le 5-benzylidène-7-(*tert*-butoxycarbonyl-amino)-5,7,8,9-tétrahydro-benzocycloheptèn-6-one préparé à l'exemple 4 que l'on hydrogène en présence de palladium à 5% sur charbon pour obtenir le 5-benzyl-7-(*tert-*butoxycarbonyl-amino)-5,7,8,9-tétrahydro-benzocycloheptèn-6-one.

On obtient le chlorhydrate correspondant en déprotégeant la fonction amine selon l'exemple 2 en utilisant 25 mg (68 µmoles) de 5-benzyl-7-(*tert-*butoxycarbonyl-amino)-5,7,8,9-tétrahydro-benzocycloheptèn-6-one et du chlorure d'hydrogène 2.2 M dans de l'éther diéthylique (0.5 mL) dans du dioxane (0.5 mL). On obtient 18 mg (86%) de cristaux incolores de chlorhydrate de 7-amino-5-benzyl-5,7,8,9-tétrahydro-benzocycloheptèn-6-one.

Point de fusion: 220-222°C

RMN ¹H (CD₃OD): 7.25-7.16 (m, 9 Har); 4.56 (dd, H-C(5)); 4.24 (dd, H-C(7)); 3.62 (dd, Ha-C(1')); 3.26 (ddd, Ha-C(9)); 3.22 (dd, Hb-C(1')); 2.85 (ddd, Hb-C(9)); 2.55 (dddd, Ha-C(8)); 1.65 (dddd, Hb-C(8)); J(1'a,1'b)=13.8 Hz, J(1'a,5) Hz, J(1'b,5)=5.8 Hz, J(7,8a)=7.4 Hz, J(7,8b)=11.4 Hz, J(8a,8b)=12.8 Hz, J(8a,9a)=2.8 Hz, J(8a,9b)=8.6 Hz, J(8b,9a)=9.8 Hz, J(8b,9b)=3.0 Hz, J(9a,9b)=14.6 Hz.

### Exemple 6: chlorhydrate de 5-phénylpropyl-7-amino-5,7,8,9-tétrahydro-benzocycloheptèn-6-one

On chauffe un mélange de 7-(benzyloxycarbonyl-amino)-5,7,8,9-tétrahydro-benzocycloheptèn-6-one (préparation 7) (300 mg, 0.97 mmole), de formaldéhyde aqueux à 36% (225 µL, 2.91 mmoles), et de pyrrolidine (50 µL) dans de l'acide acétique (10 mL) à 110°C sous argon pendant 5 heures. On dilue le milieu réactionnel à l'acétate d'éthyle, lave à l'hydrogénocarbonate de sodium aqueux 1 M puis à la saumure, et on sèche sur sulfate de magnésium. On évapore le solvant pour obtenir une résine jaunâtre de 5-méthylène-7-(benzyloxycarbonyl-amino)-5,7,8,9-tétrahydro-benzocycloheptèn-6-one.

Ensuite, à une suspension du complexe bromure de cuivre (I)-sulfure de diméthyle (440 mg, 2.13 mmoles) dans du tétrahydrofurane anhydre (20 mL) on ajoute goutte à goutte une solution de bromure de phényléthyl magnésium (3.1 mL, 1.3 M dans l'éther diéthylique, 4.27 mmoles) sous argon à -50°C. On agite le milieu réactionnel à -50°C pendant 45 minutes, puis on ajoute goutte à goutte une solution de 5-méthylène-7-(benzyloxycarbonyl-amino)-5,7,8,9-tétrahydro-benzocycloheptèn-6-one (0.97 mmole) dans du tétrahydrofurane aqueux (10 mL). On agite le milieu réactionnel à -40°C pendant 2 heures, on hydrolyse au chlorure d'ammonium aqueux 2 M, on extrait à l'acétate d'éthyle, lave à la saumure, et sèche sur sulfate de magnésium. On évapore le solvant et on purifie le résidu par chromatographie (cyclohexane/acétate d'éthyle, 9/1 puis 8/2) pour obtenir des cristaux incolores de 5-phénylpropyl-7-(benzyloxycarbonyl-amino)-5,7,8,9-tétrahydro-benzocycloheptèn-6-one (182 mg, 43%).

Point de fusion: 106-108°C

RMN ¹H (CDCl₃) : 7.34-7.11 (m, 14 Har); 5.67 (d,NH); 5.08 (s, OCH₂Ph); 4.58 (m, H-C(7)); 3.91 (m, H-C(5)); 3.06 (m, Ha-C(9)); 2.83 (m, Hb-C(9)); 2.70-2.60 (m, Ha-C(8),2H-C(3')); 2.37 (m, Ha-C(1')), 1.82 (m, Hb-C(1')); 1.63 (m, 2H-C(2')); 1.43 (m, Hb-C(8)).

On obtient le chlorhydrate correspondant en déprotégeant la fonction amine selon la méthode ci-dessous:
On hydrogénolyse le 5-phénylpropyl-7-(benzyloxycarbonyl-amino)-5,7,8,9-tétrahydro-benzocycloheptèn-6-one obtenu (139 mg, 0.325 mmole) dans du dioxane (20 mL) et de l'acide chlorhydrique aqueux 1 N (0.36 mL, 0.36 mmole) en présence de palladium à 5% sur charbon (7 mg) sous hydrogène (1 atm) à 40°C pendant 24 heures. On élimine le catalyseur par centrifugation et on évapore le solvant. On recristallise le composé obtenu dans du 2-propanol/éther diéthylique pour obtenir des cristaux incolores de chlorhydrate de 5-phénylpropyl-7-amino-5,7,8,9-tétrahydro-benzocycloheptèn-6-one (80 mg, 75%).

Point de fusion: 156-158°C

RMN ¹H (CD₃OD): 7.27-7.15 (m, 9 Har); 4.32 (dd, H-C(7)); 4.19 (dd,H-C(5)); 3.28 (m, Ha-C(9)); 2.95(ddd, Hb-C(9)); 2.70 (m, 2H-C(3')); 2.55 (m, Ha-C(8)); 2.34 (m, Ha-C(1')); 1.88 (m, Hb-C(1')); 1.67 (tt, 2H-C(2')); 1.61 (m, Hb-C(8)); J(1a',1b')=13.2 Hz, J(1a',2')=8.0 Hz, J(1a',5)=8.2 Hz, J(1b',2')=8.0 Hz, J(1b',5)=6.0 Hz, J(2',3')=7.2 Hz, J(7,8a)=7.2 Hz, J(7,8b)=11.8 Hz, J(8a,8b)=12.6 Hz, J(8a,9a)=2.6 Hz, J(8a,9b)=7.8 Hz, J(8b,9b)=2.6 Hz, J(9a,9b)=14.6 Hz.

En remplaçant le bromure de phényléthyl magnésium par le bromure de benzyl magnésium, on obtient de la même façon le chlorhydrate de 5-phényléthyl-7-amino-5,7,8,9-tétrahydro-benzocycloheptèn-6-one.

### Exemple 7: chlorhydrate de 7-amino-4-phényl-5,7,8,9-tétrahydro-benzocycloheptèn-6-one

On chauffe un mélange de 7-(*tert*-butoxycarbonyl-amino)-4-bromo-5,7,8,9-tétrahydro-benzocycloheptèn-6-one (90 mg, 0.25 mmole), acide phénylboronique (35 mg, 0.28 mmole), fluorure de césium (86 mg, 0.56 mmole) et de tétrakistriphénylphosphine palladium (30 mg, 0.025 mmole) dans du 1,2-diméthoxyléthane anhydre (3 mL) sous argon à 85°C pendant 5 heures. On dilue le milieu réactionnel à l'acétate d'éthyle, on lave à la saumure et on sèche sur sulfate de magnésium. On évapore le solvant et on purifie le résidu par chromatographie (cyclohexane/acétate d'éthyle, 8/2) pour obtenir des cristaux incolores de 7-(*tert*-butoxycarbonyl-amino)-4-phényl-5,7,8,9-tétrahydro-benzocycloheptèn-6-one (70 mg, 78%).

Point de fusion: 173-174°C

RMN ¹H (CDCl₃): 7.45-7.17 (m, 8 Har); 5.44 (d, NH); 4.55 (ddd,H-C(7)); 3.79 (d, Ha-C(5)); 3.72 (d, Hb-C(5)); 3.07 (m, Ha-C(9)); 2.96 (m, Hb-C(9)); 2.67 (m, Ha-C(8)); 1.54 (m, Hb-C(8)); 1.42 (s, *t*Bu); J(7,NH)=ca 6.7 Hz, J(5a,5b)=15.5 Hz, J(7,8a)=7.8 Hz, J(7,8b)=11.8 Hz, J(8a,9a)=3.6 Hz, J(8a,9b)=9.5 Hz, J(8b,9a)=8.4 Hz, J(8a,9b)=3.9 Hz, J(9a,9b)=15.0 Hz.

On obtient le chlorhydrate correspondant en déprotégeant la fonction amine selon l'exemple 2 en utilisant 50 mg (0,14 mmole) de 7-(*tert*-butoxycarbonyl-amino)-4-phényl-5,7,8,9-tétrahydro-benzocycloheptèn-6-one et du chlorure d'hydrogène 2.2 M dans de l'éther diéthylique (0.5 mL) dans du dioxane (0.5 mL). On obtient 30 mg (73%) de cristaux incolores de chlorhydrate de 7-amino-4-phényl-5,7,8,9-tétrahydro-benzocycloheptèn-6-one.

Point de fusion: 222°C

RMN ¹H (CD₃OD): 7.41 (m, 5 Har); 7.28 (m, 2 Har); 7.20 (m, 1 Har); 4.39 (dd, H-C(7)); 3.99 (d, Ha-C(5)); 3.77 (d, Hb-C(5)); 3.32 (m, Ha-C(9)); 3.13 (ddd, Hb-C(9)); 2.60 (m, Ha-C(8)); 1.76 (m, Hb-C(8)); J(5a,5b)=14.4 Hz, J(7,8a)=7.2 Hz, J(7,8b)=12.4 Hz, J(8a,8b)=13.2 Hz, J(8a,9a)=2.7 Hz, J(8a,9b)=8.5 Hz, J(8b,9a)=10.6 Hz, J(8b,9b)=3.0 Hz, J(9a,9b)=15.3 Hz.

### Exemple 8: chlorhydrate de 5-benzylsulfanyl-7-amino-5,7,8,9-tétrahydro-benzocycloheptèn-6-one

A une solution de 5,6,8,9-tétrahydro-benzocycloheptèn-7-one (préparation 3) (3.07 g, 19 mmoles), de triéthylamine (3.7 mL, 27 mmoles) dans du toluène anhydre (20 mL) on ajoute goutte à goutte du trifluorométhanesulfonate de triéthylsilyle (4.9 mL, 23 mmoles) à température ambiante sous argon. On chauffe le milieu réactionnel à 90°C pendant 2 heures et on dilue au cyclohexane, on lave à la saumure. On sèche la phase organique sur sulfate de magnésium et on évapore pour obtenir un éther d'énol silylé utilisé sans autre purification.

On agite cet éther d'énol silylé (19 mmoles), de l'acétate de palladium (II) (430 mg, 1.9 mmoles) dans du diméthylsulfoxyde anhydre (25 mL) à température ambiante sous oxygène (1 atm) pendant 20 heures. On dilue le milieu réactionnel à l'éther diéthylique, lave à la saumure et sèche sur sulfate de magnésium. On évapore le solvant et on distille le résidu à 82°C sous 0.05 Torr pour obtenir le 5,6-dihydro-benzocycloheptèn-7-one (2.59 g, 85%).

Ensuite, on agite une solution de 5,6-dihydro-benzocycloheptèn-7-one (0.5 g, 3.16 mmoles), d'isopropoxyde de titane (IV) (1.9 mL, 6.33 mmoles) et d'ammoniac saturé dans l'éthanol (10 mL) sous argon pendant une nuit. On ajoute ensuite du borohydrure de sodium (132 mg, 3.48 mmoles), et on agite le milieu réactionnel à température ambiante pendant encore 1 heure. On évapore les solvants et on dilue le résidu à l'acétate d'éthyle, et on ajoute de l'hydroxyde d'ammonium aqueux 1 N (20 mL). On filtre le précipité inorganique obtenu et on lave par un mélange 1/1 d'acétate d'éthyle et d'hydroxyde d'ammonium aqueux 1 N (3 x 20 mL). On sépare la phase organique et on extrait la phase aqueuse restante à l'acétate d'éthyle (3 x 20 mL). On sèche les extraits organiques combinés sur sulfate de magnésium et on concentre à sec pour obtenir l'amine éthylénique.

On agite un mélange de cette amine éthylénique, de dicarbonate de di-*tert*-butyle (1.4 g, 6.33 mmoles), et de carbonate de sodium (370 mg, 3.48 mmoles) dans du méthanol (6 mL) sous argon pendant 3 heures. On élimine les solides par filtration, évapore le solvant, et on lave le solide obtenu à l'eau (3 x 20 mL) et à l'éther isopropylique froid (3 x 20 mL) pour obtenir des cristaux incolores de 7-(*tert-*butoxycarbonyl-amino)-6,7-dihydro-5*H*-benzocycloheptène (625 mg, 76%) correspondant à un dérivé de formule (V):

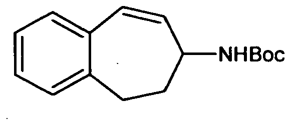

Point de fusion: 146-148°C

RMN ¹H (CDCl₃): 7.18-7.11 (m, 4 Har); 6.44 (dd, H-C(9)); 5.76 (dd,H-C(8)); 4.69 (d, NH); 4.49 (s, H-C(7)); 2.84 (m, Ha-C(5)); 2.73 (m, Hb-C(5)); 2.04 (m, 2H-C(6)); 1.46 (s, *t*Bu); J(7,8)=4.0 Hz, J(7,9)=1.9 Hz, J(8,9)=12.3 Hz. A une solution de 7-(*tert*-butoxycarbonyl-amino)-6,7-dihydro-5*H*-benzocycloheptène (400 mg, 1.54 mmole) dans du dichlorométhane anhydre (20 mL) on ajoute par portions de l'acide 3-chloroperoxybenzoïque (610 mg, 2.46 mmoles) sous argon à 0°C. On agite le milieu réactionnel à température ambiante pendant une nuit, et on dilue à l'acétate d'éthyle (10 mL), on ajoute du thiosulfate de sodium pentahydraté (3.05 g, 12,3 mmoles) et de l'hydrogénocarbonate de sodium aqueux 1 N et on agite à température ambiante pendant 1 heure. On lave la solution organique successivement à l'hydrogénocarbonate de sodium aqueux 1 N et à la saumure, et on sèche sur sulfate de magnésium. On évapore le solvant, et on recristallise le solide obtenu dans du 2-propanol pour obtenir des cristaux incolores de 7-(*tert-*butoxycarbonyl-amino)-5,6-époxy-6,7,8,9-tétrahydro-5*H-*benzocycloheptène (390 mg, 90%).

Point de fusion: 170-172°C

RMN ¹H (CDCl₃): 7.50 (m, 1 Har); 7.23 (m, 2 Har); 7.08 (m, 1 Har); 4.98 (d, NH); 4.36 (m, H-C(7)); 3.99 (d,H-C(5)); 3.68 (d, H-C(6)); 2.84 (dd, Ha-C(9)); 2.63 (dd, Hb-C(9)); 1.95 (m, Ha-C(8)); 1.69 (m, Hb-C(8)); 1.47 (s, *t*Bu); J(5,6)=4.2 Hz, J(6,7)=2.4 Hz, J(NH,7)=9.0 Hz, J(7,8a)=4.4 Hz, J(7,8b)=10.6 Hz, J(8a,8b)=13.4 Hz, J(8a,9a)=8.8 Hz, J(8b,9b)=10.4 Hz, J(9a,9b)=15.4 Hz.

On agite une solution de 7-(*tert*-butoxycarbonyl-amino)-5,6-époxy-6,7,8,9-tétrahydro-5*H*-benzocycloheptène (52 mg, 0.19 mmole), de triéthylamine (63 µL, 0.45 mmole) et de benzènethiol (30 µL, 0.23 mmole) dans de l'éthanol (1 mL) sous argon à température ambiante pendant une nuit. On dilue le milieu réactionnel à l'eau et on filtre le précipité obtenu, on lave à l'éther isopropylique (3 fois) pour obtenir le dérivé correspondant à la formule (VI) 5-benzylsulfanyl-7-(*tert*-butoxycarbonyl-amino)-6,7,8,9-tétrahydro-5H-benzocycloheptèn-6-ol (60 mg, 80%).

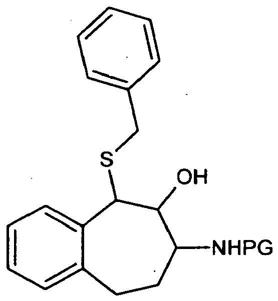

RMN ¹H (CDCl₃): 7.32-7.15 (m, 6 Har); 7.15 (t, J=7.3 Hz, 2 Har); 6.98 (d, J=7.3 Hz, 1 Har); 4.99 (d, J=6.0 Hz, NH); 4.22 (m,H-C(7)); 4.09 (m, H-C(6)); 3.94 (d, J=6.0 Hz, H-C(5)); 3.71 (d, J=13.8 Hz, Ha-(SBn)); 3.56 (d, J=13.8 Hz, Hb-(SBn)); 3.38 (t, J=13.8 Hz, Ha-C(9)); 2.66 (m, Hb-C(9)); 2.00 (m, Ha-C(8)); 1.46 (m, Hb-C(8)); 1.45 (s, *t*Bu).

Pour obtenir le 5-benzylsulfânyl-7-(*tert-*butoxycarbonyl-amino)-5,7,8,9-tétrahydrobenzocycloheptèn-6-one (40 mg), on reproduit la synthèse décrite selon la préparation 6 en utilisant le 5-benzylsulfanyl-7-(*tert-*butoxycarbonyl-amino)-6,7,8,9-tétrahydro-5*H*-benzocycloheptèn-6-ol (40 mg, 0. 1 mmole) et le periodinane de Dess-Martin (47 mg, 0.11 mmole) dans 5 mL de CH₂Cl_{2.}

RMN ¹H (CDCl₃): 7.30-7.03 (m, 9 Har) ; 5. 34 (m, NH, H-C(7)); 4.42 (s, H-C(5)); 3.72 (s, SCH₂Ph); 3.53 (m, Ha-C(9)); 2.74 (m, Hb-C(9)); 2.53 (m, Ha-C(8)); 1.43 (m, Hb-C(8)); 1.42 (s, tBu).

La déprotection de l'amine se fait de la même manière que dans l'exemple 2 en utilisant 40 mg (0.1 mmole) de 5-benzylsulfanyl-7-(*tert*-butoxycarbonyl-amino)-5,7,8,9-tétrahydrobenzocycloheptèn-6-one, du chlorure d'hydrogène 2.2 M dans de l'éther diéthylique (0.5 mL) dans du dioxane (0.5 mL), pour obtenir des cristaux incolores de chlorhydrate de 5-benzylsulfanyl-7-amino-5,7,8,9-tétrahydro-benzocycloheptèn-6-one (20 mg, 66%).

Point de fusion: 176-180°C

RMN ¹H (CDCl₃): 7.36-7.09 (m, 9 Har); 5.18 (dd, H-C(7)); 4.60 (s, H-C(5)); 3.86 (d, Ha-C(SBn)); 3.79 (d, Hb-C(SBn)); 3.64 (dd, Ha-C(9)); 2.98 (ddd, Hb-C(9)); 2.54 (m, Ha-C(8));1.76 (m,Hb-C(8)); J(SBn)=13.7 Hz, J(7,8a)=6.0 Hz, J(7,8b)=12.5 Hz, J(8a,8b)=13.5 Hz, J(8a,9a)=2.0 Hz, J(8a,9b)=8.0 Hz, J(8b,9a)=12.0 Hz, J(8b,9b)=2.0 Hz, J(9a,9b)=15.6 Hz.

### Exemple 9: chlorhydrate de 7-amino-1-bromo-5,7,8,9-tétrahydro-benzocycloheptèn-6-one

On reproduit les synthèses selon les préparations 2, 3, 4, et 6 mais en utilisant au départ le composé 1-bromo-2,3-bis-bromométhyl-benzène (préparation 1) au lieu du dibromo-*o*-xylène. On obtient le 7-(*tert-*butoxycarbonyl-amino)-1-bromo-5,7,8,9-tétrahydro-benzocycloheptèn-6-one.

La déprotection de l'amine se fait de la même manière que dans l'exemple 2 en utilisant 25 mg (71 µmoles) de 7-(*tert*-butoxycarbonyl-amino)-1-bromo-5,7,8,9-tétrahydro-benzocycloheptèn-6-one, du chlorure d'hydrogène 2.2 M dans de l'éther diéthylique (0.5 mL) dans du dioxane (0.5 mL), pour obtenir des cristaux incolores de chlorhydrate de 7-amino-1-bromo-5,7,8,9-tétrahydro-benzocycloheptèn-6-one (15 mg, 74%).

Point de fusion: 117-118°C

RMN ¹H (CD₃OD) : 7.57 (d, J=8.2 Hz, 1 Har); 7.25 (d, J=7.7 Hz, 1 Har); 7.14 (t, J=8.0 Hz, 1 Har);4.29 (dd, H-C(7)); 4.20 (d, Ha-C(5)); 3.79 (d, Hb-C(5)); 3.46 (m, Ha-C(9)); 3.35 (m, Hb-C(9)); 2.54 (m, Ha-C(8)); 1.71 (m,Hb-C(8)); J(5a,5b)=15.7 Hz, J(7,8a)=7.6 Hz, J(7,8b)=12.1 Hz, J(8a,8b)=13.4 Hz, J(8a,9a)=9.8 Hz, J(8a,9b)=3.6 Hz, J(8b,9a)=3.8 Hz, J(8b,9b)=8.4 Hz, J(9a,9b)=15.4 Hz.

### Activité inhibitrice d'AP-N

Les composés selon l'invention ont fait l'objet d'essais démontrant leur intérêt comme substances actives en thérapeutique.

Ils ont en particulier été testés comme inhibiteur d'AP-N.

A cet effet, différentes molécules inhibitrices d'AP-N connues et selon l'invention ont été testées par mesure de leur constante d'inhibition Ki, non seulement sur l'AP-N mais également sur la leucine aminopeptidase cytosolique (LAPc) et sur la leukotriène A₄ hydrolase recombinante humaine (LTA₄H) afin de démontrer la sélectivité dés molécules. La LTA₄H possède une activité de type aminopeptidasique similaire à l'AP-N et une spécificité de substrat très voisine. Il est important que les molécules inhibitrices d'AP-N n'inhibent pas la LTA₄H ni les aminopeptidases qui contiennent une unité co-catalytique comme la LAPc.

Les enzymes LAPc issues de reins de bovins et AP-N issues de reins de porcs sont commercialisées par Sigma Chemical Corporation. L'AP-N est purifiée sous forme soluble selon le procédé décrit par Wacker,H., Lecky,P., Fischer E.H., Stein, E.A. Helv. Chim. Acta, 1971, 54, 473-484.

On a réalisé des tests par spectrophotométrie avec la para-nitroanilide de L-leucine comme substrat pour LAPc (Kₘ=2 mM), AP-N (Kₘ=0.2 mM), et la para-nitroanilide d'alanine pour LTA₄H (Kₘ=2 mM). Les études cinétiques sont réalisées à 30°C et les réactions débutent par l'addition de l'enzyme dans 1 ml du milieu d'essai: LAPc, 5 unités dans 10 mM Tris-HCl, 0.1 mM ZnCl₂, 5 mM MnCl₂, 1 M KCl, pH=8.0; AP-N, 25 mUnités dans 10 mM Tris-HCl, pH=7.5 et LTA₄H, 5 µg dans 10 mM Tris-HCl, 0.1 mM KCl, pH=7.5.

On suit la libération de la para-nitroaniline (ε=10 800 M⁻¹cm⁻¹) à 405 nm pour déterminer les vitesses initiales. On mesure les constantes d'inhibition Ki par la méthode de Dixon (Segel,H. in Enzyme Kinetics, 1975, pp 109-144).

Les résultats sont indiqués dans le tableau III ci-dessous montrant les valeurs Ki(M) de différentes molécules inhibitrices évaluées pour les 3 types d'aminopeptidases testées.

Toutes les molécules inhibitrices sont évaluées sous la forme chlorhydrate et en mélange racémique.

On utilise les molécules inhibitrices synthétisées selon les exemples 2, 3, 6, 7, 8, 9.

A titre comparatif, on utilise des molécules connues dans la littérature pour leur activité inhibitrice d'AP-N:
La 2-amino-3-tétralone correspondant à l'exemple 10 (comparatif) 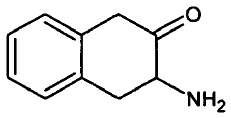
Et la bestatine correspondant à l'exemple 11 (comparatif)

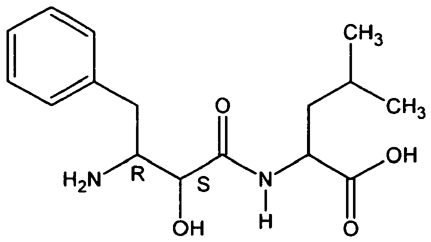

**Tableau III**

| | Ki (M) | | |
|---|---|---|---|
| Molécules | AP-N (EC3.4.11.2) | LTA4H (EC3.3.2.6) | LAPc (EC3.4.11.1) |
| EX 2 (inv.) | 1 x10⁻⁶ | >>10⁻³ | >>10⁻³ |
| EX 3 (inv.) | 3 x10⁻⁷ | >>10⁻³ | >>10⁻³ |
| EX 6 (inv.) | 1.2 x10⁻⁵ | >>10⁻³ | >>10⁻³ |
| EX 7 (inv.) | 7 x10⁻⁹ | >>10⁻³ | 2 x10⁻⁵ |
| EX 8 (inv.) | 8 x10⁻⁸ | >>10⁻³ | >>10⁻³ |
| EX 9 (inv.) | 2 x10⁻⁸ | >>10⁻³ | >>10⁻³ |
| EX 10 (comp.) | 5 x10⁻⁷ | >>10⁻³ | 1.2 x10⁻⁴ |
| EX 11 (comp.) | 3 x10⁻⁶ | 5 x10⁻⁷ | 5 x10⁻¹⁰ |

Les résultats du tableau III montrent que seules les molécules selon l'invention présentent une grande sélectivité vis à vis de l'AP-N.

### Stabilité en solution aqueuse

Pour ce test, on utilise la molécule selon l'exemple 2 de l'invention et à titre comparatif la 2-amino-3-tétralone (Ex 10) comme inhibiteurs de l'AP-N.

Les essais enzymatiques sont réalisés à 25°C dans 20 mM de Tris-HCl, pH=7.5 avec 0.2 mM de para-nitroanilide de L-leucine comme substrat, dans un volume de réaction total de 1 mL. On suit la libération de la para-nitroaniline (ε=10 800 M⁻¹cm⁻¹) à 405 nm. La réaction commence par l'addition de 3 milliunités d'AP-N issues de reins de porcs. Dans ces conditions expérimentales, on suit la cinétique linéaire pendant au moins 10 heures. On compare l'évolution de la concentration de para-nitroaniline entre un échantillon A sans inhibiteur, un échantillon B comprenant 1 µM de 2-amino-3-tétralone (Ex 10) et un échantillon C comprenant 200 µM de chlorhydrate de 7-amino-5,7,8,9-tétrahydro-benzocycloheptèn-6-one (Ex 2). On observe pour l'échantillon B, une inhibition de 40% avec la 2-amino-3-tétralone pendant la première heure. Au bout de 4 heures de test, l'activité enzymatique redevient identique à celle du contrôle. Au contraire, pour l'échantillon C, on observe une inhibition de 50% pendant tout le test, ce qui indique une stabilité de la molécule de chlorhydrate de 7-amino-5,7,8,9-tétrahydro-benzocycloheptèn-6-one après 10 heures de test.

On obtient les mêmes résultats avec tous les autres composés de l'invention testés.

Les composés selon l'invention comportant un cycle à 7 atomes de carbone sont donc des molécules beaucoup plus stables en solution aqueuse que la molécule de 2-amino-3-tétralone qui comporte un cycle à 6 atomes de carbone, déjà connue comme inhibiteur d'AP-N mais présentant l'inconvénient d'être peu stable.

## Revendications

1. Composé répondant à la formule générale (I) : 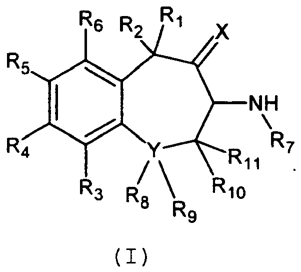 dans laquelle,
R₁ représente un atome d'hydrogène, de fluor, de chlore, de brome, un radical (C₁-C₆)alkyle, un radical (C₁-C₆) (cycloalkyl) alkyle, un radical (C₁-C₆) (hétérocycloalkyl) alkyle, un radical (C₁-C₆) aralkyle, un radical (C₁-C₆) hétéroaralkyle, un radical (C₁-C₆) alcoxy, un radical (C₁-C₆) aralkyloxy, un radical (C₁-C₆)alkylthio, un radical (C₁-C₆) aralkylthio;
R₂ représente un atome d'hydrogène, de fluor, de chlore, de brome, un radical (C₁-C₆)alkyle, un radical (C₁-C₆) (cycloalkyl)alkyle, un radical (C₁-C₆) (hétérocycloalkyl) alkyle, un radical (C₁-C₆) aralkyle, un radical (C₁-C₆)hétéroaralkyle; R₁ et R₂ pouvant former ensemble un cycle carboné non substitué ou substitué ou un hétérocycle non substitué ou substitué; ou R₁ pouvant être lié au cycle heptène par une double liaison, R₂ étant alors absent;
R₃, R₄, R₅ et R₆, identiques ou différents, représentent indépendamment l'un de l'autre un atome d'hydrogène, de fluor, de chlore, de brome, un radical (C₁-C₆)alkyle, un radical (C₁-C₆)(cycloalkyl)alkyle, un radical (C₁-C₆) (hétérocycloalkyl) alkyle, un radical polyfluoro(C₁-C₆)alkyle, un radical (C₁-C₆)aralkyle, un radical (C₁-C₆)hétéroaralkyle, un radical (C₁-C₆)alcoxy, un groupe aryle ou hétéroaryle; R₃ et R₄, R₄ et R₅, R₅ et R₆ indépendamment les uns des autres pouvant former ensemble un radical méthylènedioxy joignant les atomes de carbone adjacents ou un cycle carboné aromatique non substitué ou substitué ou un hétérocycle aromatique non substitué ou substitué;
R₇ représente un atome d'hydrogène, un radical (C₁-C₆)alkyle;
X est un atome d'oxygène, un atome de soufre, un radical imine N-R₁₂, un radical oxime N-O-R₁₃, dans lesquels R₁₂ et R₁₃ représentent un atome d'hydrogène, un radical (C₁-C₆)alkyle, un radical (C₁-C₆) (cycloalkyl)alkyle, un radical (C₁-C₆) (hétérocycloalkyl)alkyle, un radical (C₁-C₆)aralkyle, un radical (C₁-C₆)hétéroaralkyle;
Y est un atome de carbone; un atome d'azote, R₈ ou R₉ étant alors absent; un atome d'oxygène, un atome de soufre R₈ et R₉ étant alors absents;
R₈ et R₁₀, identiques ou différents, représentent indépendamment l'un de l'autre un atome d'hydrogène, de fluor, de chlore, de brome, un radical (C₁-C₆)alkyle, un radical (C₁-C₆) (cycloalkyl)alkyle, un radical (C₁-C₆)(hétérocycloalkyl)alkyle, un radical (C₁-C₆)aralkyle, un radical (C₁-C₆)hétéroaralkyle, un radical (C₁-C₆)alcoxy, un radical (C₁-C₆)aralkyloxy, un radical (C₁-C₆)alkylthio, un radical (C₁-C₆)aralkylthio;
R₉ et R₁₁, identiques ou différents, représentent indépendamment l'un de l'autre un atome d'hydrogène, de fluor, de chlore, de brome, un radical (C₁-C₆)alkyle, un radical (C₁-C₆)(cycloalkyl)alkyle, un radical (C₁-C₆)(hétérocycloalkyl)alkyle, un radical (C₁-C₆)aralkyle, un radical (C₁-C₆)hétéroaralkyle, un radical (C₁-C₆)alcoxy, un radical (C₁-C₆)aralkyloxy, un radical (C₁-C₆)alkylthio, un radical (C₁-C₆) aralkylthio, R₉ et R₁₁ pouvant former ensemble un cycle carboné non substitué ou substitué ou un hétérocycle non substitué ou substitué ou former une double liaison avec les deux atomes de carbone adjacents du cycle heptène;
ses isomères optiques et géométriques et leurs mélanges, ainsi que ses sels d'addition avec des acides minéraux et organiques, à l'exception du composé pour lequel R₄, R₅ et R₆ représentent un radical méthoxy, R₁, R₂, R₃, R₇, R₈, R₉, R₁₀, R₁₁ représentent un atome d'hydrogène, X représente un atome d'oxygène et Y représente un atome de carbone.

2. Composé selon la revendication 1, **caractérisé en ce que** R₁ représente un atome d'hydrogène, un atome de fluor, un radical (CH₂)ₙPh, un radical S(CH₂)ₙPh, n variant de 1 à 6;
ses isomères optiques et géométriques et leurs mélanges, ainsi que ses sels d'addition avec des acides minéraux et organiques.

3. Composé selon l'une quelconque des revendications précédentes, **caractérisé en ce que** R₂ est un atome d'hydrogène;
ses isomères optiques et géométriques et leurs mélanges, ainsi que ses sels d'addition avec des acides minéraux et organiques.

4. Composé selon l'une quelconque des revendications précédentes, **caractérisé en ce que** X est un atome d'oxygène;
ses isomères optiques et géométriques et leurs mélanges, ainsi que ses sels d'addition avec des acides minéraux et organiques.

5. Composé selon l'une quelconque des revendications précédentes, **caractérisé en ce que** Y est un atome de carbone;
ses isomères optiques et géométriques et leurs mélanges, ainsi que ses sels d'addition avec des acides minéraux et organiques.

6. Composé selon l'une quelconque des revendications précédentes, **caractérisé en ce que** R₃, R₄, R₅ et R₆ identiques ou différents, représentent indépendamment l'un de l'autre un atome d'hydrogène, un atome de brome, un radical phényle, ou R₃ et R₄, R₄ et R₅, R₅ et R₆ indépendamment les uns des autres forment ensemble un cycle carboné aromatique non substitué ou substitué;
ses isomères optiques et géométriques et leurs mélanges, ainsi que ses sels d'addition avec des acides minéraux et organiques.

7. Composé selon l'une quelconque des revendications précédentes, **caractérisé en ce que** R₇ est un atome d'hydrogène;
ses isomères optiques et géométriques et leurs mélanges, ainsi que ses sels d'addition avec des acides minéraux et organiques.

8. Composé selon l'une quelconque des revendications précédentes, **caractérisé en ce que** simultanément Y est un atome de carbone et R₈, R₉, R₁₀, et R₁₁ sont des atomes d'hydrogène;
ses isomères optiques et géométriques et leurs mélanges, ainsi que ses sels d'addition avec des acides minéraux et organiques.

9. Composé selon l'une quelconque des revendications précédentes, **caractérisé en ce que** R₂ et R₇ sont simultanément un atome d'hydrogène, X est un atome d'oxygène et Y est un atome de carbone.

10. Composé selon la revendication précédente, **caractérisé en ce que** R₁ est un atome d'hydrogène, un atome de fluor, un radical benzylthio, un radical (CH₂)ₙPh, où n=1-5.

11. Composé selon la revendication précédente, **caractérisé en ce que** R₃, R₄, R₅, R₆ sont simultanément un atome d'hydrogène.

12. Composé selon la revendication 10, **caractérisé en ce que** R₄ et R₅ sont un atome d'hydrogène, et R₃ et R₆ représentent indépendamment l'un de l'autre un atome d'hydrogène, un atome de brome, un radical phényle, à la condition que R₃ et R₆ ne soient pas simultanément un atome d'hydrogène.

13. Composé selon la revendication 10, **caractérisé en ce que** R₃ et R₆ sont un atome d'hydrogène, et R₄ et R₅ représentent indépendamment l'un de l'autre un atome d'hydrogène, un atome de brome, un radical phényle, à la condition que R₄ et R₅ ne soient pas simultanément un atome d'hydrogène.

14. Composé selon la revendication 10, **caractérisé en ce que** R₃ et R₄, R₅ et R₆ indépendamment les uns des autres forment ensemble un cycle carboné aromatique, non substitué ou substitué, joignant les atomes de carbone adjacents.

15. Composé selon la revendication 1, **caractérisé en ce qu'**il répond à la formule (Ia): 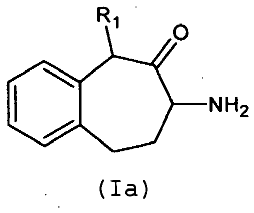 dans laquelle R₁ représente un atome d'hydrogène, un atome de fluor, le radical CH₂Ph, le radical (CH₂)₂Ph, le radical (CH₂)₃Ph, le radical (CH₂)₄Ph, le radical (CH₂)₅Ph, le radical S-CH₂Ph, le radical =CH-Ph; ainsi que ses sels d'addition avec des acides minéraux et organiques.

16. Composé selon la revendication 1, **caractérisé en ce qu'**il est choisi parmi les composés de formules (Ib) et (Ic) suivantes; ainsi que ses sels d'addition avec des acides minéraux et organiques:

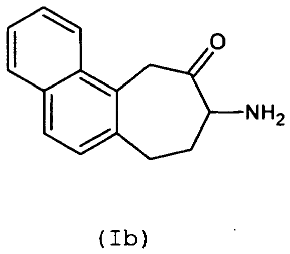

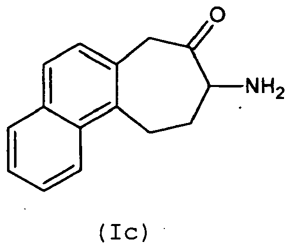

17. Composé selon la revendication 1, **caractérisé en ce qu'**il répond à la formule (Id): 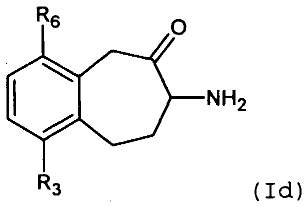 dans laquelle
R₃ est un atome d'hydrogène et R₆ est un radical phényle; R₃ est un atome d'hydrogène et R₆ est un atome de brome; R₃ est un radical phényle et R₆ est un atome d'hydrogène; R₃ est un atome de brome et R₆ est un atome d'hydrogène; R₃ est un radical phényle et R₆ est un atome de brome; R₃ est un atome de brome et R₆ est un radical phényle; R₃ et R₆ sont respectivement un radical phényle; R₃ et R₆ sont respectivement un atome de brome;
ainsi que ses sels d'addition avec des acides minéraux et organiques.

18. Composé selon la revendication 1, **caractérisé en ce qu'**il répond à la formule (Ie): 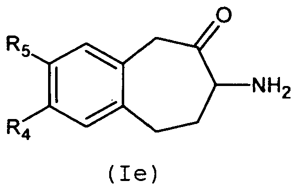 dans laquelle
R₄ est un atome d'hydrogène et R₅ est un radical phényle; R₄ est un atome d'hydrogène et R₅ est un atome de brome, R₄ est un radical phényle et R₅ est un atome d'hydrogène, R₄ est un atome de brome et R₅ est un atome d'hydrogène, R₄ et R₅ sont respectivement un radical phényle, R₄ et R₅ sont respectivement un atome de brome; ainsi que ses sels d'addition avec des acides minéraux et organiques.

19. Procédé pour la préparation d'un composé de formule (I) selon la revendication 1, où R₁ représente un atome d'hydrogène, un atome de fluor, un radical (CH₂)ₙPh, le radical =CH-Ph, R₂ est un atome d'hydrogène ou absent, R₇, R₈, R₉, R₁₀, et R₁₁ sont des atomes d'hydrogène; X est un atome d'oxygène, un radical NOH, Y est un atome de carbone, R₃, R₄, R₅, R₆ ont la signification déjà indiquée; et ses sels, **caractérisé en ce que**
1) on introduit sur le composé de formule générale (II) 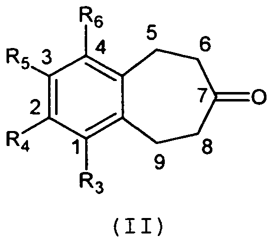 une fonction amine protégée -NHPG en position 7 par réaction de la fonction cétone, où PG est un groupe protecteur, et en position 6 une fonction cétone lorsque X est un atome d'oxygène ou une fonction cétone-oxime lorsque X est le radical NOH pour former un dérivé de formule générale (III) 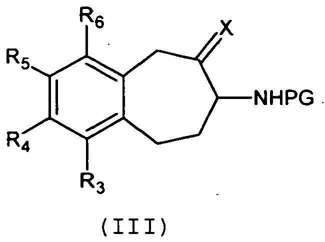
2) lorsque R₁ n'est pas un atome d'hydrogène, on introduit la fonction correspondant à R₁ en position 5 pour former un dérivé de formule générale (IV) 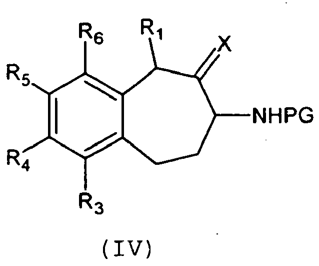
3) on déprotège la fonction amine NH-PG par clivage du groupe PG.

20. Procédé pour la préparation d'un composé de formule (I) selon la revendication 1, où R₁ représente un radical S(CH₂)ₙPh, R₂ est un atome d'hydrogène, R₇, R₈, R₉, R₁₀, et R₁₁ sont des atomes d'hydrogène; X est un atome d'oxygène, Y est un atome de carbone, R₃, R₄, R₅, R₆ ont la signification déjà indiquée; et ses sels, **caractérisé en ce que**
1) on forme sur le composé de formule générale (II) 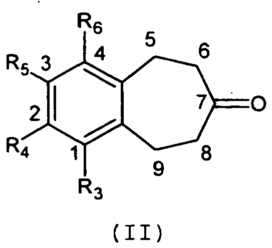 une double liaison entre les positions 5 et 6
2) on introduit une fonction amine protégée -NHPG en position 7 par réaction de la fonction cétone, où PG est un groupe protecteur pour former un dérivé de formule générale (V) 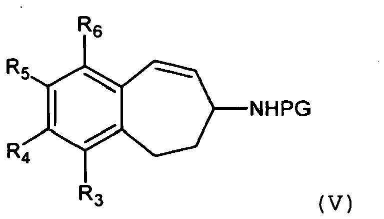
3) on oxyde la double liaison pour former une fonction époxyde liant les atomes de carbone en positions 5 et 6
4) on introduit le radical S(CH₂)ₙPh en position 5 pour former un dérivé de formule générale (VI) 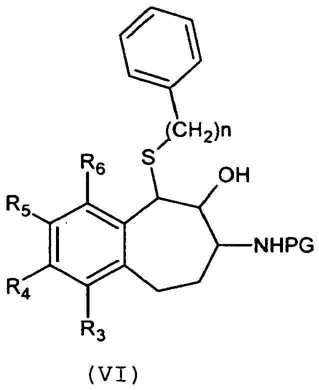
5) on oxyde la fonction alcool du dérivé obtenu
6) on déprotège la fonction amine NH-PG par clivage du groupe PG.

21. Composition pharmaceutique, **caractérisée en ce qu'**elle contient à titre de principe actif un composé de formule (I) selon l'une quelconque des revendications 1 à 18, ou un de ses sels d'addition avec des acides minéraux et organiques pharmaceutiquement acceptables, étant entendu que le composé pour lequel R₄, R₅ et R₆ représentent un radical méthoxy, R₁, R₂, R₃, R₇, R₈, R₉, R₁₀, R₁₁ représentent un atome d'hydrogène, X représente un atome d'oxygène et Y représente un atome de carbone n'est pas exclu.

22. Composition pharmaceutique selon la revendication 21, **caractérisée en ce que** le principe actif est mélangé à au moins un excipient pharmaceutiquement acceptable.

23. Utilisation d'un composé de formule (I) selon l'une quelconque des revendications 1 à 18, étant entendu que le composé pour lequel R₄, R₅ et R₆ représentent un radical méthoxy, R₁, R₂, R₃, R₇, R₈, R₉, R₁₀, R₁₁ représentent un atome d'hydrogène, X représente un atome d'oxygène et Y représente un atome de carbone n'est pas exclu, pour la préparation d'un médicament destiné au traitement des cancers.

24. Utilisation d'un composé de formule (I) selon l'une quelconque des revendications 1 à 18, étant entendu que le composé pour lequel R₄, R₅ et R₆ représentent un radical méthoxy, R₁, R₂, R₃, R₇, R₈, R₉, R₁₀, R₁₁ représentent un atome d'hydrogène, X représente un atome d'oxygène et Y représente un atome de carbone n'est pas exclu, pour la préparation d'un médicament destiné au traitement des tumeurs, et notamment pour traiter et prévenir les maladies impliquant l'inhibition des métalloprotéases, plus particulièrement l'Aminopeptidase-N.

## Claims

1. A compound of the general formula (I): 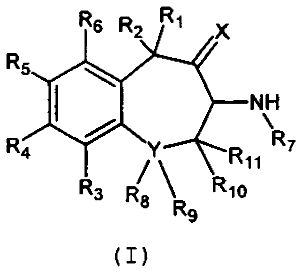 wherein,
R₁ represents an hydrogen, fluorine, chlorine, bromine atom, a (C₁-C₆)alkyl radical, a (C₁-C₆) (cycloalkyl) alkyl radical, a (C₁-C₆) (heterocycloalkyl)alkyl radical, a (C₁-C₆)aralkyl radical, a (C₁-C₆)heteroaralkyl radical, a (C₁-C₆)alkoxy radical, a (C₁-C₆)aralkyloxy radical, a (C₁-C₆)alkylthio radical, a (C₁-C₆)aralkylthio radical;
R₂ represents an hydrogen, fluorine, chlorine, bromine atom, a (C₁-C₆)alkyl radical, a (C₁-C₆) (cycloalkyl)alkyl radical, a (C₁-C₆) (heterocycloalkyl)alkyl radical, a (C₁-C₆)aralkyl radical, a (C₁-C₆)heteroaralkyl radical; where R₁ and R₂ can form together an unsubstituted or substituted carbon ring or an unsubstituted or substituted heterocycle; or R₁ can be bonded to the heptene ring through a double bond, R₂ being then absent;
R₃, R₄, R₅ and R₆, the same or different, represent independently of each other a hydrogen, fluorine, chlorine, bromine atom, a (C₁-C₆)alkyl radical, a (C₁-C₆)(cycloalkyl)alkyl radical, a (C₁-C₆) (heterocycloalkyl)alkyl radical, a polyfluoro(C₁-C₆)alkyl radical, a (C₁-C₆)aralkyl radical, a (C₁-C₆)heteroaralkyl radical, a (C₁-C₆)alkoxy radical, an aryl or heteroaryl group; R₃ and R₄, R₄ and R₅, R₅ and R₆ independently of each other can form together a methylenedioxy radical joining the adjacent carbon atoms or an unsubstituted or substituted aromatic carbon ring or an unsubstituted or substituted aromatic heterocycle;
R₇ represents a hydrogen atom, a (C₁-C₆)alkyl radical;
X is an oxygen atom, a sulphur atom, an imine radical N-R₁₂, an oxime radical N-O-R₁₃, wherein R₁₂ and R₁₃ represent a hydrogen atom, a (C₁-C₆)alkyl radical, a (C₁-C₆)(cycloalkyl)alkyl radical, a (C₁-C₆)(heterocycloalkyl)alkyl radical, a (C₁-C₆)aralkyl radical, a (C₁-C₆)heteroaralkyl radical;
Y is a carbon atom; a nitrogen atom, R₈ or R₉ being then absent; an oxygen atom, a sulphur atom, R₈ and R₉ being then absent;
R₈ and R₁₀, the same or different, represent independently of each other a hydrogen, fluorine, chlorine, bromine atom, a (C₁-C₆)alkyl radical, a (C₁-C₆)(cycloalkyl)alkyl radical, a (C₁-C₆)(heterocycloalkyl)alkyl radical, a (C₁-C₆)aralkyl radical, a (C₁-C₆)heteroaralkyl radical, a (C₁-C₆)alkoxy radical, a (C₁-C₆)aralkyloxy radical, a (C₁-C₆)alkylthio radical, a (C₁-C₆)aralkylthio radical;
R₉ and R₁₁, the same or different, represent independently of each other a hydrogen, fluorine, chlorine, bromine atom, a (C₁-C₆)alkyl radical, a (C₁-C₆)(cycloalkyl)alkyl radical, a (C₁-C₆)(heterocycloalkyl)alkyl radical, a (C₁-C₆)aralkyl radical, a (C₁-C₆)heteroaralkyl radical, a (C₁-C₆)alkoxy radical, a (C₁-C₆)aralkyloxy radical, a (C₁-C₆)alkylthio radical, a (C₁-C₆)aralkylthio radical, where R₉ and R₁₁ can form together an unsubstituted or substituted carbon ring or an unsubstituted or substituted heterocycle or form a double bond with the two adjacent carbon atoms of the heptene ring;
optical and geometrical isomers thereof and mixtures thereof, as well as inorganic and organic acid addition salts thereof, except for the compound wherein R₄, R₅ and R₆ represent a methoxy radical, R₁, R₂, R₃, R₇, R₈, R₉, R_{10,} R₁₁ represent a hydrogen atom, X represents an oxygen atom and Y represents a carbon atom.

2. The compound according to claim 1, **characterised in that** R₁ represents a hydrogen atom, a fluorine atom, a (CH₂)ₙPh radical, a S(CH₂)ₙPh radical, n ranging from 1 to 6;
optical and geometrical isomers thereof and mixtures thereof, as well as inorganic and organic acid addition salts thereof.

3. The compound according to any of the preceding claims, **characterised in that** R₂ is a hydrogen atom;
optical and geometrical isomers thereof and mixtures thereof, as well as inorganic and organic acid addition salts thereof.

4. The compound according to any of the preceding claims, **characterised in that** X is an oxygen atom;
optical and geometrical isomers thereof and mixtures thereof, as well as inorganic and organic acid addition salts thereof.

5. The compound according to any of the preceding claims, **characterised in that** Y is a carbon atom;
optical and geometrical isomers thereof and mixtures thereof, as well as inorganic and organic acid addition salts thereof.

6. The compound according to any of the preceding claims, **characterised in that** R₃, R₄, R₅ and R₆, the same or different, represent independently of each other, a hydrogen atom, a bromine atom, a phenyl radical, or R₃ and R₄, R₄ and R₅, R₅ and R₆ independently of each other form together an unsubstituted or substituted aromatic carbon ring;
optical and geometrical isomers thereof and mixtures thereof, as well as inorganic and organic acid addition salts thereof.

7. The compound according to any of the preceding claims, **characterised in that** R₇ is a hydrogen atom;
optical and geometrical isomers thereof and mixtures thereof, as well as inorganic and organic acid addition salts thereof.

8. The compound according to any of the preceding claims, **characterised in that** simultaneously Y is a carbon atom and R₈, R₉, R₁₀ and R₁₁ are hydrogen atoms;
optical and geometrical isomers thereof and mixtures thereof, as well as inorganic and organic acid addition salts thereof.

9. The compound according to any of the preceding claims, **characterised in that** R₂ and R₇ are simultaneously a hydrogen atom, X is an oxygen atom and Y is a carbon atom.

10. The compound according to the preceding claim, **characterised in that** R₁ is a hydrogen atom, a fluorine atom, a benzylthio radical, a (CH₂)ₙPh radical,
where n=1-5.

11. The compound according to the preceding claim, **characterised in that** R₃, R₄, R₅, R₆ are simultaneously a hydrogen atom.

12. The compound according to claim 10, **characterised in that** R₄ and R₅ are a hydrogen atom, and R₃ and R₆ represent independently of each other a hydrogen atom, a bromine atom, a phenyl radical, with the proviso that R₃ and R₆ are not simultaneously a hydrogen atom.

13. The compound according to claim 10, **characterised in that** R₃ and R₆ are a hydrogen atom, and R₄ and R₅ represent independently of each other a hydrogen atom, a bromine atom, a phenyl radical, with the proviso that R₄ and R₅ are not simultaneously a hydrogen atom.

14. The compound according to claim 10, **characterised in that** R₃ and R₄, R₅ and R₆ independently of each other, form together an unsubstituted or substituted aromatic carbon ring, joining the adjacent carbon atoms.

15. The compound according to claim 1, **characterised in that** it is of the formula (Ia): 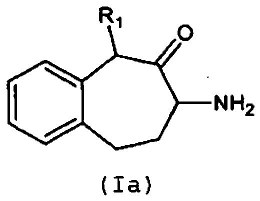 wherein R₁ represents a hydrogen atom, a fluorine atom, the CH₂Ph radical, the (CH₂)₂Ph radical, the (CH₂)₃Ph radical, the (CH₂)₄Ph radical, the (CH₂)₅Ph radical, the S-CH₂Ph radical, the =CH-Ph radical; as well as inorganic and organic acid addition salts thereof.

16. The compound according to claim 1, **characterised in that** it is selected from the compounds of the following formulae (Ib) and (Ic); as well as inorganic and organic acid addition salts thereof:

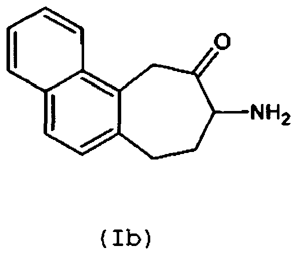

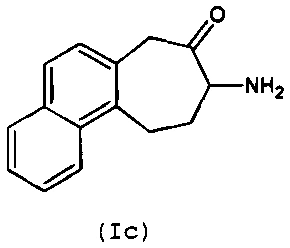

17. The compound according to claim 1, **characterised in that** it is of the formula (Id): 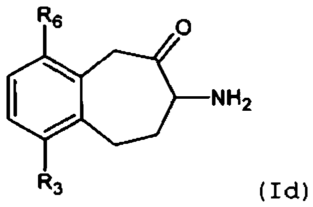 wherein
R₃ is a hydrogen atom and R₆ is a phenyl radical; R₃ is a hydrogen atom and R₆ is a bromine atom; R₃ is a phenyl radical and R₆ is a hydrogen atom; R₃ is a bromine atom and R₆ is a hydrogen atom; R₃ is a phenyl radical and R₆ is a bromine atom; R₃ is a bromine atom and R₆ is a phenyl radical; R₃ and R₆ are a phenyl radical respectively; R₃ and R₆ are a bromine atom respectively;
as well as inorganic and organic acid addition salts thereof.

18. The compound according to claim 1, **characterised in that** it is of the formula (Ie): 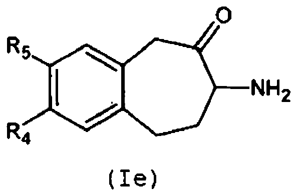 wherein
R₄ is a hydrogen atom and R₅ is a phenyl radical; R₄ is a hydrogen atom and R₅ is a bromine atom, R₄ is a phenyl radical and R₅ is a hydrogen atom, R₄ is a bromine atom and R₅ is a hydrogen atom, R₄ and R₅ are a phenyl radical respectively, R₄ and R₅ are a bromine atom respectively; as well as inorganic and organic acid addition salts thereof.

19. A method for making a compound of formula (I) according to claim 1, wherein R₁ represents a hydrogen atom, a fluorine atom, a (CH₂)ₙPh radical, the =CH-Ph radical, R₂ is a hydrogen atom or is absent, R₇, R₈, R₉, R₁₀, and R₁₁ are hydrogen atoms; X is an oxygen atom, a NOH radical, Y is a carbon atom, R₃, R₄, R₅, R₆ have the meanings already set out; and salts thereof, **characterised in that**
1) a -NHPG protected amine function is introduced onto the compound of the general formula (II) 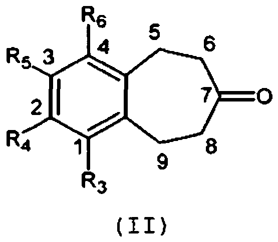 at the 7-position by reacting the ketone function, wherein PG is a protecting group, and at the 6-position, a ketone function is introduced when X is an oxygen atom or a ketone-oxime function is introduced when X is the NOH radical to form a derivative of the general formula (III) 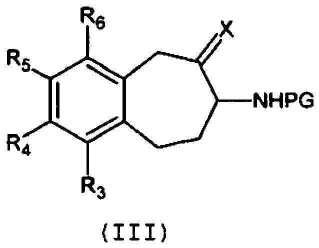
2) when R₁ is not a hydrogen atom, the function corresponding to R₁ is introduced at the 5-position to form a derivative of the general formula (IV) 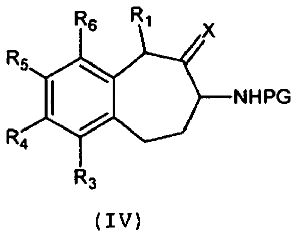
3) the amine NH-PG function is deprotected by cleaving the PG group.

20. The method for making a compound of formula (I) according to claim 1, wherein R₁ represents a S(CH₂)ₙPh radical, R₂ is a hydrogen atom, R₇, R₆, R₉, R₁₀ and R₁₁ are hydrogen atoms; X is an oxygen atom, Y is a carbon atom, R₃, R₄, R₅, R₆ have the meanings already set out;
and salts thereof, **characterised in that**
1) a double bond is formed onto the compound of the general formula (II) 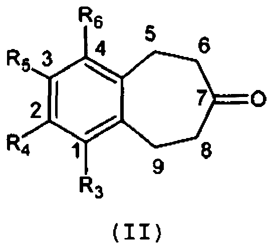 between the 5- and 6-positions
2) a -NHPG protected amine function is introduced at the 7-position by reacting the ketone function, wherein PG is a protecting group to form a derivative of the general formula (V) 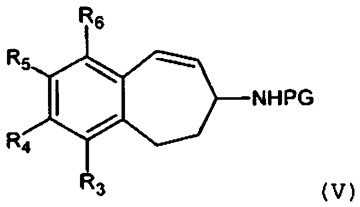
3) the double bond is oxidized to form an epoxide function bonding together the carbon atoms at the 5- and 6-positions
4) the S(CH₂)ₙPh radical is introduced at the 5-position to form a derivative of the general formula (VI) 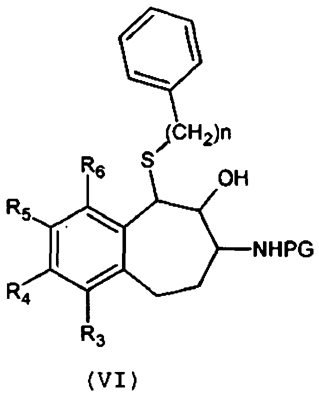
5) the alcohol function of the resulting derivative is oxidized
6) the NH-PG amine function is deprotected by cleaving the PG group.

21. A pharmaceutical composition, **characterised in that** it contains as an active principle, a compound of the formula (I) according to any of claims 1 to 18, or pharmaceutically acceptable inorganic and organic acid addition salts thereof, with the proviso that the compound wherein R₄, R₅ and R₆ represent a methoxy radical, R₁, R₂, R₃, R₇, R₈, R₉, R₁₀ and R₁₁ represent a hydrogen atom, X represents an oxygen atom and Y represents a carbon atom is not excluded.

22. The pharmaceutical composition according to claim 21, **characterised in that** the active principle is mixed with at least a pharmaceutically acceptable excipient.

23. A use of a compound of the formula (I) according to any of claims 1 to 18, with the proviso that the compound wherein R₄, R₅ and R₆ represent a methoxy radical, R₁, R₂, R₃, R₇, R₈, R₉, R₁₀ and R₁₁ represent a hydrogen atom, X represents an oxygen atom and Y represents a carbon atom is not excluded, for making a drug for treating cancers.

24. The use of a compound of the formula (I) according to any of claims 1 to 18, with the proviso that the compound wherein R₄, R₅ and R₆ represent a methoxy radical, R₁, R₂, R₃, R₇, R₈, R₉, R₁₀ and R₁₁ represent a hydrogen atom, X represents an oxygen atom and Y represents a carbon atom is not excluded, for making a drug for treating tumours, and in particular for treating and preventing diseases involving inhibition of metalloproteases, more particularly the Aminopeptidase-N.

## Patentansprüche

1. Verbindung, die der allgemeinen Formel (I) entspricht: 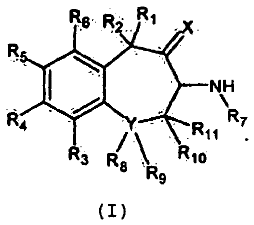 worin:
R₁ ein Wasserstoff-, Fluor-, Chlor-, Bromatom, ein (C₁-C₆)Alkylradikal, ein (C1- C6)(Cycloalkyl)alkylradikal, ein (C₁-C₆) (Heterocycloalkyl)alkylradikal, ein (C₁-C₆) Aralkylradikal, ein (C₁-C₆) Heteroaralkylradikal, ein (C₁-C₆) Alcoxyradikal, ein (C₁-C₆) Aralkyloxyradikal, ein (C₁-C₆) Alkylthioradikal, ein (C₁-C₆) Aralkylthioradikal darstellt;
R₂ ein Wasserstoff-, Fluor-, Chlor-, Bromatom, ein(C₁-C₆)Alkylradikal, ein (C1- C6)(Cycloalkyl)alkylradikal, ein (C₁-C₆) (Heterocycloalkyl)alkylradikal, ein (C₁-C₆) Aralkylradikal, ein (C₁-C₆) Heteroaralkylradikal darstellt; R₁ und R₂ zusammen einen nicht substituierten oder substituierten Kohlenstoffzyklus oder einen nicht substituierten oder substituierten Heterozyklus bilden können; oder R₁ mit dem Heptenzyklus durch eine doppelte Bindung verbunden sein kann, wobei R₂ dann nicht vorhanden ist;
R₃, R₄, Rund R₆, identisch oder verschieden, unabhängig von einander ein Wasserstoff-, Fluor-, Chlor-, Bromatom, ein (C₁-C₆)Alkylradikal, ein (C₁-C₆) (Cycloalkyl) alkylradikal, ein (C₁-C₆) (Heterocycloalkyl)alkylradikal, ein Polyfluor(C₁-C₆)alkylradikal, ein (C₁-C₆) Aralkylradikal, ein (C₁-C₆) Heteroralkylradikal, ein(C₁-C₆) Alkoxyradikal, eine Aryl- oder Heteroarylgruppe darstellen; R₃ und R₄, R₄ und R₅, R₅ und R₆ unabhängig voneinander zusammen ein Methylendioxyradikal bilden können, das die benachbarten Kohlenstoffatome miteinander verbindet, oder einen nicht substituierten oder substituierten aromatischen Kohlenstoffzyklus oder einen nicht substituierten oder substituierten aromatischen Heterozyklus;
R₇ ein Wasserstoffatom, ein (C₁-C₆)Alkylradikal darstellt;
X ein Sauerstoffatom, ein Schwefelatom, ein N-R₁₂-Iminradikal, ein N-O-R₁₃-Oximradiakl ist, in denen R₁₂ und R₁₃ ein Wasserstoffatom, ein (C₁-C₆) Alkylradikal, ein (C₁-C₆) (Cycloalkyl)alkylradikal, ein (C₁-C₆) (Heterocycloalkyl)alkylradikal, ein (C₁-C₆) Aralkylradikal, ein (C₁-C₆) Heteroaralkylradikal darstellen;
Y ein Kohlenstoffatom ist; ein Stickstoffatom, wobei R₈ oder R₉ dann nicht vorhanden sind; ein Sauerstoffatom, ein Schwefelatom, wobei R₈ und R₉ dann nicht vorhanden sind;
R₈ und R₁₀, identisch oder verschieden, unabhängig von einander ein Wasserstoff-, Fluor-, Chlor-, Bromatom, ein (C₁-C₆)Alkylradikal, ein (C₁-C₆)(Cycloalkyl)alkylradikal, ein (C₁-C₆) (Heterocycloalkyl)alkylradikal, ein (C₁-C₆) Aralkylradikal, ein (C₁-C₆) Heteroralkylradikal, ein (C₁-C₆) Alkoxyradikal, ein (C₁-C₆)Aralkyloxyradikal, ein (C₁-C₆) Alkylthioradikal, ein (C₁-C₆) Aralkylthioradikal darstellen;
R₉ und R₁₁, identisch oder verschieden, unabhängig von einander ein Wasserstoff-, Fluor-, Chlor-, Bromatom, ein (C₁-C₆)Alkylradikal, ein (C₁-C₆)(Cycloalkyl)alkylradikal, ein (C₁-C₆) (Heterocycloalkyl)alkylradikal, ein (C₁-C₆) Aralkylradikal, ein (C₁-C₆) Heteroralkylradikal, ein (C₁-C₆) Alkoxyradikal, ein (C₁-C₆)Aralkyloxyradikal, ein (C₁-C₆) Alkylthioradikal, ein (C₁-C₆) Aralkylthioradikal darstellen, wobei R₉ et R₁₁ zusammen einen nicht substituierten oder substituierten Kohlenstoffzyklus oder einen nicht substituierten oder substituierten Heterozyklus bilden können oder mit den zwei benachbarten Kohlenstoffatomen des Heptenzyklus eine doppelte Bindung bilden können;
ihre optischen und geometrischen Isomere und ihre Mischungen, ebenso wie ihre Additionssalze mit Mineral- und organischen Säuren, mit Ausnahme der Verbindung, für die R₄, R₅ und R₆ ein Methoxyradikal darstellen, R₁, R₂, R₃, R₇, R₈, R₉, R₁₀, R₁₁ ein Wasserstoffatom darstellen, Z ein Sauerstoffatom und Y ein Kohlenstoffatom darstellt.

2. Verbindung nach Anspruch 1, **dadurch gekennzeichnet, dass** R₁ ein Wasserstoffatom, ein Fluoratom, ein (CH₂)ₙPh-Radikal, ein S(CH₂)ₙPh-Radikal darstellt, wobei n von 1 bis 6 variiert;
ihre optischen und geometrischen Isomere und ihre Mischungen, ebenso wie ihre Additionssalze mit Mineral- und organischen Säuren.

3. Verbindung nach einem der vorhergehenden Ansprüche, **dadurch gekennzeichnet, dass** R₂ ein Wasserstoffatom ist;
seine optischen und geometrischen Isomere und seine Mischungen, ebenso wie seine Additionssalze mit Mineral- und organischen Säuren.

4. Verbindung nach einem der vorhergehenden Ansprüche, **dadurch gekennzeichnet, dass** X ein Sauerstoffatom ist;
seine optischen und geometrischen Isomere und seine Mischungen, ebenso wie seine Additionssalze mit Mineral- und organischen Säuren.

5. Verbindung nach einem der vorhergehenden Ansprüche, **dadurch gekennzeichnet, dass** Y ein Kohlenstoffatom ist;
seine optischen und geometrischen Isomere und seine Mischungen, ebenso wie seine Additionssalze mit Mineral- und organischen Säuren.

6. Verbindung nach einem der vorhergehenden Ansprüche, **dadurch gekennzeichnet, dass** R₃, R₄, R₅ und R₆ identisch oder verschieden, unabhängig von einander ein Wasserstoffatom, ein Bromatom, ein Phenylradikal darstellen, wobei R₃ und R₄, R₄ und R₅, R₅ und R₆ unabhängig voneinander zusammen einen nicht substituierten oder substituierten aromatischen Kohlenstoffzyklus bilden;
seine optischen und geometrischen Isomere und seine Mischungen, ebenso wie seine Additionssalze mit Mineral- und organischen Säuren.

7. Verbindung nach einem der vorhergehenden Ansprüche, **dadurch gekennzeichnet, dass** R₇ ein Wasserstoffatom ist;
seine optischen und geometrischen Isomere und seine Mischungen, ebenso wie seine Additionssalze mit Mineral- und organischen Säuren.

8. Verbindung nach einem der vorhergehenden Ansprüche, **dadurch gekennzeichnet, dass** gleichzeitig Y ein Kohlenstoffatom ist und R₈, R₉, R₁₀ und R₁₁ Wasserstoffatome sind;
ihre optischen und geometrischen Isomere und ihre Mischungen, ebenso wie ihre Additionssalze mit Mineral- und organischen Säuren.

9. Verbindung nach einem der vorhergehenden Ansprüche, **dadurch gekennzeichnet, dass** R₂ und R₇ gleichzeitig ein Wasserstoffatom, X ein Sauerstoffatom und y ein Kohlenstoffatom ist.

10. Verbindung nach dem vorhergehenden Anspruch, **dadurch gekennzeichnet, dass** R₁ ein Wasserstoffatom, ein Fluoratom, ein Benzylthioradikal ein (CH₂)ₙPh-Radikal ist, wobei n=1-5.

11. Verbindung nach dem vorhergehenden Anspruch, **dadurch gekennzeichnet, dass** R₃, R₄, R₅, R₆ gleichzeitig ein Wasserstoffatom sind.

12. Verbindung nach Anspruch 10, **dadurch gekennzeichnet, dass** R₄ und R₅ ein Wasserstoffatom sind und R₃ und R₆ unabhängig voneinander ein Wasserstoffatom, ein Bromatom, ein Phenylradikal darstellen, unter der Bedingung, dass R₃ und R₆ nicht gleichzeitig ein Wasserstoffatom sind.

13. Verbindung nach Anspruch 10, **dadurch gekennzeichnet, dass** R₃ und R₆ ein Wasserstoffatom sind und R₄ und R₅ unabhängig voneinander ein Wasserstoffatom, ein Bromatom, ein Phenylradikal darstellen, unter der Bedingung, dass R₄ und R₅ nicht gleichzeitig ein Wasserstoffatom sind.

14. Verbindung nach Anspruch 10, **dadurch gekennzeichnet, dass** R₃ und R₄, R₅ und R₆ unabhängig voneinander zusammen einen nicht substituierten oder substituierten aromatischen Kohlenstoffzyklus bilden, der die benachbarten Kohlenstoffatome verbindet.

15. Verbindung nach Anspruch 1, **dadurch gekennzeichnet, dass** sie der Formel (Ia) entspricht: 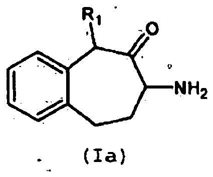 wobei R₁ ein Wasserstoffatom, ein Fluoratom, das CH₂Ph-Radikal, das (CH₂)₂Ph-Radikal, das (CH₂)₃Ph-Radikal, das (CH₂)₄Ph-Radikal, das (CH₂)₅Ph-Radikal, das S-CH₂Ph-Radikal, das =CH-Ph-Radikal darstellt; ebenso wie ihre Additionssalze mit Mineral- und organischen Säuren.

16. Verbindung nach Anspruch 1, **dadurch gekennzeichnet, dass** sie ausgewählt ist aus den Verbindungen der folgenden Formeln (Ib) und Ic); ebenso wie ihren Additionssalzen mit Mineral- und organischen Säuren:

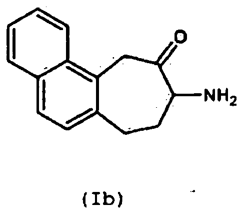

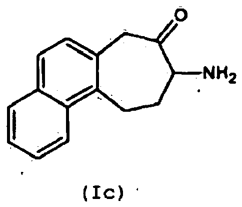

17. Verbindung nach Anspruch 1, **dadurch gekennzeichnet, dass** sie der Formel (Id) entspricht: 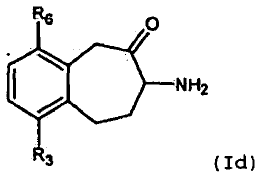 worin:
R₃ ein Wasserstoffatom und R₆ ein Phenylradikal ist; R₃ Wasserstoffatom und R₆ ein Bromatom ist; R₃ ein Phenylradikal und R₆ ein Wasserstoffatom ist; R₃ ein Bromatom und R₆ ein Wasserstoffatom ist; R₃ ein Phenylradikal und R₆ ein Bromatom ist; R₃ ein Bromatom und R₆ ein Phenylradikal ist; R₃ und R₆ jeweils ein Phenylradikal sind; R₃ und R₆ jeweils ein Bromatom sind;
ebenso wie ihre Additionssalze mit Mineral- und organischen Säuren.

18. Verbindung nach Anspruch 1, **dadurch gekennzeichnet, dass** sie der Formel (Ie) entspricht: 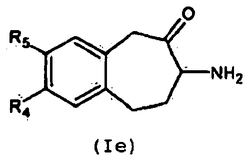 worin:
R₄ ein Wasserstoffatom und R₅ ein Phenylradikal ist; R₄ ein Wasserstoffatom und R₅ ein Bromatom ist, R₄ ein Phenylradikal und R₅ ein Wasserstoffatom ist, R₄ ein Bromatom und R₅ ein Wasserstoffatom ist, R₄ und R₅ jeweils ein Phenylradikal sind, R₄ und R₅ jeweils ein Bromatom sind; ebenso wie ihre Additionssalze mit Mineral- und organischen Säuren.

19. Verfahren zur Herstellung einer Verbindung der Formel (I) nach Anspruch 1, wobei R₁ ein Wasserstoffatom, ein Fluoratom, ein (CH₂)ₙPh-Radikal, das =CH-Ph-Radikal darstellt, R₂ ein Wasserstoffatom oder abwesend ist, R₇, R₈, R₉, R₁₀, und R₁₁ Wasserstoffatome sind; X ein Sauerstoffatom ist, ein NOH-Radikal, Y ein Kohlenstoffatom ist, R₃, R₄, R₅, R₆ die bereits angegebene Bedeutung haben; und ihre Salze, **dadurch gekennzeichnet, dass**
1) auf der Verbindung der allgemeinen Formel (II) Folgendes eingeführt wird: 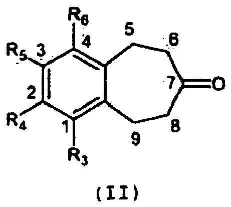 eine geschützte Aminfunktion -NHPG in der Position 7 durch Reaktion der Ketonfunktion, wobei PG eine Schutzgruppe ist, und in der Position 6 eine Ketonfunktion, wenn X ein Wasserstoffatom ist oder eine Keton-Oximfunktion ist, wenn X das NOH-Radikal ist, um ein Derivat der allgemeinen Formel (III) zu bilden: 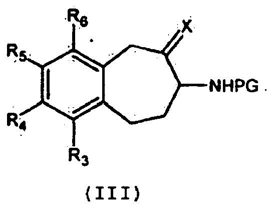
2) wenn R₁ kein Wasserstoffatom ist, die Funktion, die R₁ entspricht, in die Position 5 eingeführt wird, um ein Derivat der allgemeinen Formel (IV) zu bilden: 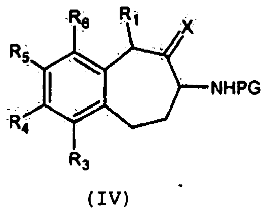
3) die Aminfunktion NH-PG durch Spaltung der PG-Gruppe entschützt wird.

20. Verfahren zur Herstellung einer Verbindung der Formel (I) nach Anspruch 1, wobei R₁ ein S(CH₂)ₙPh-Radikal darstellt, R₂ ein Wasserstoffatom ist, R₇, R₈, R₉, R₁₀ und R₁₁ Wasserstoffatome sind; X ein Sauerstoffatom ist, Y ein Kohlenstoffatom ist, R₃, R₄, R₅, R₆ die bereits angegebene Bedeutung haben; und ihre Salze, **dadurch gekennzeichnet, dass**
1) auf der Verbindung der allgemeinen Formel (II) Folgendes gebildet wird: 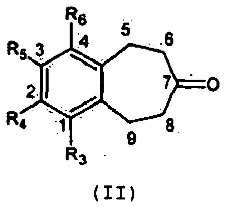 eine doppelte Verbindung zwischen den Positionen 5 und 6
2) eine geschützte Aminfunktion -NHPG in die Position 7 durch Reaktion der Ketonfunktion eingeführt wird, wobei PG eine Schutzgruppe ist, um ein Derivat der allgemeinen Formel (V) zu bilden: 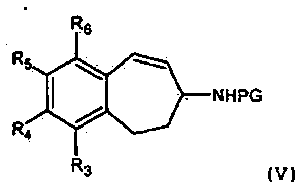
3) die doppelte Verbindung oxydiert wird, um eine Epoxydfunktion zu bilden, die die Kohlenstoffatome an den Positionen 5 und 6 verbindet
4) das S(CH₂)ₙPh-Radikal in die Position 5 eingeführt wird, um ein Derivat der allgemeinen Formel (VI) zu bilden 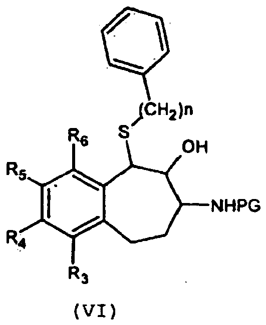
5) die Alkoholfunktion des erhaltenen Derivats oxydier wird t
6) die Aminfunktion NH-PG durch Spaltung der PG-Gruppe entschützt wird.

21. Pharmazeutische Verbindung, **dadurch gekennzeichnet, dass** sie als Wirkstoff eine Verbindung der Formel (I) nach einem der Ansprüche 1 bis 18 enthält oder eines ihrer Additionssalze mit pharmazeutisch annehmbaren Mineral- und organischen Säuren, wobei davon ausgegangen wird, dass die Verbindung, für die R₄, R₅ und R₆ ein Methoxyradikal darstellen, R₁, R₂, R₃, R₇, R₈, R₉, R₁₀, R₁₁ ein Wasserstoffatom darstellen, X ein Sauerstoffatom darstellt und Y ein Kohlenstoffatom darstellt, nicht ausgeschlossen ist.

22. Pharmazeutische Verbindung nach Anspruch 21, **dadurch gekennzeichnet, dass** der Wirkstoff mit mindestens einem pharmazeutisch annehmbaren Trägerstoff gemischt wird.

23. Verwendung einer Verbindung der Formel (I) nach einem der Ansprüche 1 bis 18, wobei davon ausgegangen wird, dass die Verbindung, für die R₄, R₅ und R₆ ein Methoxyradikal darstellen, R₁, R₂, R₃, R₇, R₈, R₉, R₁₀, R₁₁ ein Wasserstoffatom darstellen, X ein Sauerstoffatom darstellt und Y ein Kohlenstoffatom darstellt, nicht ausgeschlossen ist, für die Herstellung eines Arzneimittels für die Behandlung von Krebserkrankungen.

24. Verwendung einer Verbindung der Formel (I) nach einem der Ansprüche 1 bis 18, wobei davon ausgegangen wird, dass die Verbindung, für die R₄, R₅ und R₆ ein Methoxyradikal darstellen, R₁, R₂, R₃, R₇, R₈, R₉, R₁₀, R₁₁ ein Wasserstoffatom darstellen, X ein Sauerstoffatom darstellt und Y ein Kohlenstoffatom darstellt, nicht ausgeschlossen ist, für die Herstellung eines Arzneimittels für die Behandlung von Krebserkrankungen und besonders zur Behandlung und Vorbeugung von Krankheiten, die die Hemmung der Metalloproteasen, insbesondere der Aminopeptidase-N implizieren.
